# EUROPEAN PATENT APPLICATION

(11) **EP 2 208 785 A1**
(43) Date of publication of application: **21.07.2010**
(21) Application number: 09382003.3
(22) Date of filing: 15.01.2009
(51) Int. Cl.: C12N 15/09, C12N 15/10, C12N 15/11

(54) **Methods and kits to generate miRNA- and smallRNA-expressing vectors, and its application to develop lentiviral expression libraries**

(71) Applicant: NEWBIOTECHNIC, S.A., 41110 Bollullos de la Mitacion (Sevilla) (ES); CONSEJO SUPERIOR DE INVESTIGACIONES CIENTIFICAS, 28006 Madrid (ES)
(72) Inventor: Pintor Toro, José Antonio, Parque Cientifico y Technológico Cartuja 93 41092 Sevilla (ES); Moreno Mateos, Miguel Angel, Parque Cientifico y Technológico Cartuja 93 41092 Sevilla (ES)
(74) Representative: ABG Patentes, S.L.

(57) **Abstract**

The invention relates to novel expression systems for microRNAs (miRNAs) based on the expression of the sense and antiscnsc strand from convergent promoters. The invention also provides a method for the direct cloning of small RNAs present in an RNA preparation by the direct ligation of the small RNAs present within the preparation to vectors. Interestingly, if the vectors contain convergent promoter regions, the vectors isolated according to the method of the invention can be used for the direct expression of the small RNA without the need of using the DNA encoding for the small RNA precursor molecule.

SEQUENCE LISTING

<110>  NEWBIOTECHNIC, S.A.

<120>  METHODS AND KITS TO GENERATE miRNA- AND smallRNA-EXPRESSING
       VECTORS, AND ITS APPLICATION TO DEVELOP LENTIVIRAL EXPRESSION
       LIBRARIES

<130>  P4221EP00

<140>  EP 09382003.3
<141>  2009/01/15

<160>  8

<170>  PatentIn version 3.5

<210>  1
<211>  41
<212>  DNA
<213>  Artificial sequence

<220>
<223>  Primer for removing the BbvII site in the luciferase gene
       sequence

<400>  1
gccattctac ccactcgagg acgggaccgc cggcgagcag c                          41


<210>  2
<211>  28
<212>  DNA
<213>  Artificial sequence

<220>
<223>  3' adapter


<220>
<221>  adenosine 5' phosphorimidazolide
<222>  (1)..(1)

<220>
<221>  dideoxycytosine
<222>  (28)..(28)

<400>  2
ctttttgtc ttccgcccgt cgccatac                                          28


<210>  3
<211>  27
<212>  DNA
<213>  Artificial sequence

<220>
<223>  5' adapter


<220>

<221>  RNA
<222>  (21)..(27)

<400>  3
cggctcaact ctctggaaga ctcaaag                                          27


<210>  4
<211>  18
<212>  DNA
<213>  Artificial sequence

<220>
<223>  Reverse transcription primer

<400>  4
gtatgcccgc tcagtgaa                                                    18


<210>  5
<211>  18
<212>  DNA
<213>  Artificial sequence

<220>
<223>  PCR Primer

<400>  5
gtatgcccgc tcagtgaa                                                    18


<210>  6
<211>  18
<212>  DNA
<213>  Artificial sequence

<220>
<223>  PCR primer

<400>  6
cggctcaact ctctggaa                                                    18


<210>  7
<211>  24
<212>  DNA
<213>  Artificial sequence

<220>
<223>  H1 primer

<400>  7
ctgggaaatc accataaacg tgaa                                             24


<210>  8
<211>  26
<212>  DNA
<213>  Artificial sequence

<220>

<223>  U6 primer

<400>  8
gcttaccgta acttgaaagt atttcg                                          26

## Description

### TECHNICAL FIELD OF THE INVENTION

The invention relates to the field of molecular biology and, more particularly, to novel nucleic acids suitable for the expression of miRNA in a cell wherein the sense and antisense strand of the miRNA are produced by transcription of a template DNA under the control of separate and preferably convergent promoters. The invention also relates to libraries comprising these polynucleotides and methods to identify genes involved in cellular processes using such libraries.

### BACKGROUND ART

The human genome project has enabled scientists with a powerful tool to study the function of individual genes through suitable high-throughput genetic screens. Genetic screens that aim to identify gene function through inactivation of a gene by classical methods have shown severe limitations due to the high cost and the technical difficulties. Recently various approaches have been developed to study the effects of gene suppression in mammalian cells, emphasizing the RNA interference (RNAi) and the microRNAs (miRNAs) technologies. In fact, RNAi libraries have now been generated by a number of commercial and academic laboratories, and come in many formats. Fundamentally, library formats can be divided in two types: collection of vector-based shRNA expression vectors and libraries of siRNAs. shRNA libraries may in turn be classified by the type of vector used, such as non-viral (plasmid), retroviral, adenoviral or lentiviral. Libraries based on retroviral or lentiviral vectors can provide long-term and stable gene silencing, as the vectors used are able to integrate into genomic DNA and are thus copied along with cellular DNA. Lentiviral libraries are particularly useful in studies involving cells that are difficult to transfect, such as primary cells. These libraries contributed to the identification of new oncogenes (Horvath et al., 2006, Proc Natl Acad Sci USA, 103, 17402-7; Pan et al., 2005, Cancer Res, 65, 8366-71; Zhang et al., 2006, Cancer Biol Ther, 5, 1481-6) and tumour suppressors (Kolfschoten et al., 2005, Cell, 121, 849-58; Westbrook et al., 2005, Cell, 121, 837-48) and to identify the functional role of specific proteins and genes in tumorigenesis (Collins et al., 2006, Proc Natl Acad Sci U S A, 103, 3775-80; Draviam et al., 2007, Nat Cell Biol, 9, 556-64; MacKeigan et al., 2005, Nat Cell Biol, 7, 591-600; Moffat et al., 2006, Cell, 124, 1283-98; Paddison et al., 2004, Nature, 428, 427-31).

The finding that RNA interference (RNAi) and microRNAs (miRNAs) can be exploited to suppress gene expression has led to the rapid identification of genes involved in many different biological processes through powerful loss-of function screens. While each siRNA knocks down a single gene, a miRNA may target numerous mRNAs, which could present extraordinary challenges for looking for phenotypes dependent of several genes. That means the modulation of a single miRNA could give rise to a whole complex phenotype. For instance, the overexpression of miR-10b in otherwise non-metastatic breast tumours initiates a robust invasion and metastasis (Ma et al., 2007, Nature, 449, 682-688).

miRNAs are short non coding RNA molecules, distinct from but related to small interfering RNAs. They have been identified in the genomes of a wide range of multicellular life forms as well as viruses. Mature miRNAs are 19- to 25-nucleotide-long molecules cleaved in two steps. First, a primary transcript knows as primary - miRNA (pri-miRNA) is transcribed and processed by Drosha-DGCR8 protein complex to form 70- to 100-nucleotide hairpin pre-miRNA precursors. Pre-miRNA is transported out of the nucleus and cleaved in the cytoplasm by Dicer to form a short double-stranded miRNA duplex. In a final step, this RNA duplex is loaded into the RNA-induced silencing complex (RISC), one of the strands is degraded, and the remaining strand engages in imperfect base pairing with the 3' untranslated region (3' UTR) of target mRNAs, and causes either block of translation or, less frequently, mRNA degradation (Bartel, 2004, Cell, 116, 281-97). This mechanism resembles the process of RNA interference triggered by double- stranded RNA and uses similar molecular machinery.

At present, miRNAs libraries have been used to identify miRNAs and to assay their expression pattern. Several companies have developed miRNA libraries to obtain gain of function phenotypes in different formats such as doble strand miRNA (miRIDIAN microRNA mimic from Dharmacon), expressed as pri-miRNA in a plasmid (miR-Lib from Geneservice) or a lentiviral based vector (miRexpress Human Lentiviral microRNA library from Open biosystem).

It is known to express miRNAs from plasmids encoding miRNAs that are transcribed by endogenous RNA polymerases to produce specific miRNAs when transfected into cells (Zeng et al., 2002 and W006137941A). However, a limitation of the plasmid-based miRNA expression system is that the transfection efficiencies for plasmids tend to be very low, with only approximately 50% of cells expressing RNA from the plasmid in cells that are easy to transfect. Transfection efficiencies for plasmids in primary cells are much lower, with fewer than 10% of cells typically expressing the desired RNA.

It is also known to express miRNA in target cells by direct transfection of liposomes including synthetic miRNAs (WO06137941A). However, the efficiency of transfection of synthetic liposomes is also low, which results in a very limited amount of cells expressing the desired miRNA.

The problem of low transfection efficenty has been overcome by the use of viral vectors. For instance, Silva et al. (Nature Genetics 37: 1281-88, 2005) have described extensive libraries of pri-miR-30- based retroviral expression vectors that can be used to down-regulate almost all known human (at least 28,000) and mouse (at least 25,000) genes (see RNAi Codex, a single database that curates publicly available RNAi resources, and provides the most complete access to this growing resource). Additionally, lentiviral vectors carrying miRNA libraries such as the pLemiR plasmid have been described (Open Biosystems) wherein the sequence encoding the miRNA is under the control of a CMV promoter. However, these plasmids encode for the precursor form of the miRNA (the pri-miRNA) so that activation by the cellular machinery is required before the miRNA can exert its biological effects.

Thus, there is a need in the art for systems for expression of miRNA which allow the direct cloning and expression of the small RNAs and, in particular, the while overcoming the drawbacks of the methods known in the prior art.

### SUMMARY OF THE INVENTION

The authors of the present invention provide systems for expression of miRNAs based on the transcription of both strands of a short DNA fragment flanked by two RNA polymerase III promoters, generating a duplex of RNA. This duplex is the core sequence of the full length miRNA. miRNAs produced by this system are functionally similar to the full length miRNA versions.

In a first aspect, the invention relates to a polynucleotide construct comprising a first promoter region operatively linked to a sequence encoding the sense strand of a miRNA and a second promoter region operatively linked to a sequence encoding the antisense strand of said miRNA.

In a second and third aspect, the invention relates, respectively, to a vector comprising a polynucleotide of the invention and to a host cell comprising the polynucleotide or the vector of the invention.

In a fourth aspect, the invention relates to a library comprising a plurality of polynucleotides according to the invention.

In a fifth aspect, the invention relates to a method for expressing a miRNA in a host cell comprising using the polynucleotide, vector or the library of the invention.

In a sixth aspect, the invention relates to a method for the preparation of a vector for expressing a miRNA comprising the steps of
(i) contacting a miRNA molecule with a 5' adapter and a 3' adapter
   wherein each adapter comprises a known sequence, wherein said contacting step is carried out under conditions adequate for promoting ligation of the adapters to the 5' and 3' ends of the RNA and for preventing circularization of the RNA,
(ii) reverse transcribing the 5'-adapter-RNA-3'adapter molecule obtained in step (i) using a primer which sequence is substantially complementary to the sequence of the 3' adapter,
(iii) producing a double stranded polynucleotide using as template the cDNA obtained in step (ii) and
(iv) inserting the polynucleotide obtained in step (iii) in a suitable vector
wherein convergent promoter sequences are present either within the known sequences of the 5' and 3' adapters or in the vector wherein the polynucleotide is inserted in step (iv) at positions 5' or 3 with respect of the site of the insertion.

In a seventh aspect, the invention relates to a method for preparing a library for expressing a miRNA comprising the steps of
(i) contacting a RNA preparation comprising at least one miRNA molecule having about 19-25 nucleotides in length with a 5' adapter and a 3' adapter of known sequences, wherein said contacting step is carried out under conditions adequate for promoting ligation of the adapters to the 5' and 3' ends of the RNA and for preventing circularization of the RNA,
(ii) reverse transcribing the 5'-adapter-RNA-3'adapter molecules obtained in step (i) using a primer which sequence is substantially complementary to the sequence of the 3' adapter,
(iii) producing double stranded polynucleotides using as template the cDNAs obtained in step (ii) and
(iv) inserting the polynucleotides obtained in step (iii) in a suitable vector
wherein convergent promoter sequences are present either within the known sequences of the 5' and 3' adapters or the vector wherein the polynucleotide is inserted in step (iv) at positions 5' or 3 with respect of the site of insertion.

In another aspect, the invention relates to a method for the identification of miRNAs capable of interfering in a cellular process comprising
(i) contacting a miRNA of the invention with a cell population under conditions adequate for promoting the entry of the library polynucleotides in the cell,
(ii) maintaining the cells under conditions adequate for promoting expression of the library-encoded miRNAs,
(iii) selecting those cells showing an alteration in the cellular process under study and
(iv) recovering the library polynucleotides from the cell.

In yet another aspect, the invention relates to a method for the identification of genes involved in a cellular process comprising
(i) contacting a miRNA library of the invention with a cell population under conditions adequate for promoting the entry of the library polynucleotides in the cell,
(ii) maintaining the cells under conditions adequate for promoting expression of the library-encoded miRNA,
(iii) selecting those cells showing an alteration in the cellular process under study,
(iv) recovering the library polynucleotides from the cell,
(v) sequencing the region of the library polynucleotide flanked by the promoter regions and
(vi) identifying genes showing a high degree of sequence relatedness in their 3'-UTR region with the sequence determined in step (v).

In yet another aspect, the invention relates to a method for the cloning of small RNAs comprising the steps of
(i) contacting a RNA preparation having about 19-25 nucleotides in length with a 5' adapter and a 3' adapter of known sequences, wherein said contacting step is carried out under conditions adequate for promoting ligation of the adapters to the 5' and 3' ends of the RNA and for preventing circularization of the RNA,
(ii) reverse transcribing the 5'-adapter-RNA-3'adapter molecules obtained in step (i) using a primer which sequence is substantially complementary to the sequence of the 3' adapter,
(iii) producing a double stranded polynucleotides using as template the cDNAs obtained in step (ii),
(iv) inserting the polynucleotides obtained in step (iii) in a suitable vector,
   wherein convergent promoter sequences are present either within the known sequences of the 5' and 3' adapters used in step (i) or in the vector wherein the polynucleotide is inserted in step (iv) at positions 5' or 3 with respect of the site of insertion.
   In yet another aspect, the invention relates to a method for the identification of small RNAs involved in a biological process comprising the steps of
(v) contacting a RNA preparation having about 19-25 nucleotides in length with a 5' adapter and a 3' adapter of known sequences, wherein said contacting step is carried out under conditions adequate for promoting ligation of the adapters to the 5' and 3' ends of the RNA and for preventing circularization of the RNA,
(vi) reverse transcribing the 5'-adapter-RNA-3'adapter molecules obtained in step (i) using a primer which sequence is substantially complementary to the sequence of the 3' adapter,
(vii) producing a double stranded polynucleotides using as template the cDNAs obtained in step (ii),
(viii) inserting the polynucleotides obtained in step (iii) in a suitable vector,
(ix) contacting the polynucleotides obtained in step (iv) with a cell population under conditions adequate for promoting the entry of the library polynucleotides in the cell,
(x) maintaining the cells under conditions adequate for promoting expression of the library-encoded small RNAs,
(xi) selecting those cells showing an alteration in the cellular process under study and
(xii) recovering the library polynucleotides from the cell
wherein convergent promoter sequences are present either within the known sequences of the 5' and 3' adapters used in step (i) or in the vector wherein the polynucleotide is inserted in step (iv) at positions 5' or 3 with respect of the site of insertion.

In another aspect, the invention relates to a method for the identification of genes involved in a cellular process comprising
(i) contacting a RNA preparation having about 19-25 nucleotides in length with a 5' adapter and a 3' adapter of known sequences,
   wherein said contacting step is carried out under conditions adequate for promoting ligation of the adapters to the 5' and 3' ends of the RNA and for preventing circularization of the RNA,
(ii) reverse transcribing the 5'-adapter-RNA-3'adapter molecules obtained in step (i) using a primer which sequence is substantially complementary to the sequence of the 3' adapter,
(iii) producing a double stranded polynucleotides using as template the cDNAs obtained in step (ii),
(iv) inserting the polynucleotides obtained in step (iii) in a suitable vector
(v) contacting the polynucleotides obtained in step (iv) with a cell population under conditions adequate for promoting the entry of the library polynucleotides in the cell,
(vi) maintaining the cells under conditions adequate for promoting expression of the library-encoded small RNAs,
(vii) selecting those cells showing an alteration in the cellular process under study,
(viii) recovering the library polynucleotides from the cell,
(ix) sequencing the region of the library polynucleotide flanked by the promoter regions and
(x) identifying genes showing a high degree of sequence relatedness with the sequence determined in step (v)
wherein convergent promoter sequences are present either within the known sequences of the 5' and 3' adapters used in step (i) or in the vector wherein the polynucleotide is inserted in step (iv) at positions 5' or 3 with respect of the site of insertion.

In another aspect, the invention relates to a kit for carrying out the method as defined in the invention comprising
(i) a 5' adapter comprising a first known sequence,
(ii) a 3' adapter comprising a second known sequence,
(iii) a vector comprising at least a cloning site,
(iv) a RNA ligase and
(v) a reverse transcriptase
wherein promoter sequences are present either within the first and second known sequences of the adapters or in the vector 5' and 3' with respect to the cloning site.

### BRIEF DESCRIPTION OF THE FIGURES

**Figure 1****.** Map and features of Lent H1/U6. Promoters H1 and U6 have been modified. The digestion of the vector with BbvII removes part of the sequence contained between the U6 and H1 promoters (shown in lower case) and generates terminal RNA polymerase III sites (T5) at both ends.
**Figure 2****.** Luciferase activity (arbritary units) in cotransfection experiments using different Lent H1/U6 miRNA constructions.
**Figure 3****.** mRNA levels of luciferase in the co-transfection experiment using Lent H1/U6-miRNA constructions. Renilla was used as a transfection control.
**Figure 4****.** Western blot of the cotransfection experiment using Lent H1/U6 miRNA constructs. Renilla activity was used as a transfection control in order to quantify the levels of protein.
**Figure 5****.** Quantification of the levels of PTEN protein in a western blot experiment GAPDH protein was used as a control.
**Figure 6****.** Schematic representation of the strategy to generate a miRNAs library.

### DETAILED DESCRIPTION OF THE INVENTION

The authors of the present invention have developed a system for the expression of miRNA that does not require cleavage of a precursor form. This has been achieved by the separate expression of the sense and antisense strands of the mature miRNA whereby the expression of each strand is driven by a different promoter for each strand. Surprisingly, the double promoter Lent-H1/U6 vector provides higher stability of RNA template inserts for propagation in *E.coli* which facilitates the construction of representative high complexity miRNA libraries. Moreover, this system allows the expression of the miRNA with an efficency similar to conventional polynucleotides carrying a pre-miRNA or pri-mRNA templates. In addition, the system allows the direct expression of the miRNA from a vector comprising the sense and antisense strand of the miRNA without the need of expressing a cDNA encoding the miRNA precursor, thus dispensing with the requirement for intracellular maturation for obtaining the mature functional miRNA. An additional advantage of the system lies in the possibility of cloning miRNAs or other types of small RNA from an heterlogous RNA preparation by directly cloning the miRNA into a vector of choice using conventional cloning methods for double stranded RNA. This avoids the known procedure of first identifying the miRNA based on its sequence followed by expression form the DNA encoding the precursor. The cloned miRNAs obtained in this manner can be directly tested in order to identify those leading to a particular phenotype when expressed in cells. This expression system allows developing functional screening once small RNAs are cloned to isolate and describe new small RNAs (miRNA among others) involved in different biological events.

### Polynucleotides of the invention

In a first aspect, the invention relates to a polynucleotide construct comprising a first promoter region operatively linked to a sequence encoding the sense strand of a miRNA and a second promoter region operatively linked to a sequence encoding the antisense strand of said miRNA.

As used herein, the term "promoter" is understood as a DNA sequence recognised by the transcriptional machinery to initiate transcription a downstream polynucleotide sequence. In principle, any two promoters can be used in the vector of the invention. Thus, non-limiting examples of promoters suitable for use in the present invention, include constitutive promoters such as those found in some eukaryotic viruses such as the polyoma virus, adenovirus, SV40, CMV, avian sarcoma virus, hepatitis B virus, herpes simplex virus thymidine kinase promoter, retroviral LTR regions as well as promoters present in eukaryotic genes such as the metalothioneine gene promoter, the immunoglobulin promoter, actin promoter, EF-1alpha promoter as well as inducible promoters wherein expression of the downstream gene requires addition of a substance or an exogenous signal to the culture such as the tetracycline promoter, NFKappaB/ UV light, Cre/lox, heat shock promoters, regulatable RNA polymerase II promoters described in WO/2006/135436 as well as tissue-specific promoters, such as the PSA promoter described in WO2006012221. In a preferred embodiment, the promoters are RNA polymerase III promoters which function in constitutive manner. RNA pol III promoters appear in a limited number of genes such as the 5S ARN, ARNt, ARN 7SL and ARNsn U6 genes. RNA pol III promoters differ from other promoters in that they do not require an intragenic sequence but require 5' sequences comprising a TATA box at positions -34 and -24, a proximal sequence element or PSE located between -66 y -47 and, in some cases, a distal sequence element o DSE) at positions -265 and -149. In a preferred embodiment, the RNA pol III promoters are the promoters found in the H1 and U6 genes of human or murine origin. In a still more preferred embodiment, the promoters are two U6 promoters of human or murine origin, a murine U6 promoter and a H1 human promoter or a U6 human promoter and a murine H1 promoter.

The term miRNA, as used herein, is understood as a double stranded non-coding RNA sequence which is capable of interacting with the 3'-untranslated region of a mRNA thus preventing its translation.

The terms "microRNA" or "miRNA" used interchangeably herein, are endogenous RNAs, some of which are known to regulate the expression of protein-coding genes at the posttranscriptional level. As used herein, the term "microRNA" refers to any type of micro- interfering RNA, including but not limited to, endogenous microRNA and artificial microRNA. Typically, endogenous microRNA are small RNAs encoded in the genome which are capable of modulating the productive utilization of mRNA. A mature miRNA is a single-stranded RNA molecule of about 21-23 nucleotides in length which is complementary to a target sequence, and hybridizes to the target RNA sequence to inhibit its translation. miRNAs themselves are encoded by genes that are transcribed from DNA but not translated into protein (non-coding RNA); instead they are processed from primary transcripts known as pri-miRNA to short stem-loop structures called pre-miRNA and finally to functional miRNA. Mature miRNA molecules are partially complementary to one or more messenger RNA (mRNA) molecules, and their main function is to down-regulate gene expression. MicroRNA sequences have been described in publications such as, Lim, et al., Genes and Development, 17, p. 991-1008 (2003), Lim et al Science 299, 1540 (2003), Lee and Ambros Science, 294, 862 (2001), Lau et al., Science 294, 858-861 (2001), Lagos-Quintana et al, Current Biology, 12, 735-739 (2002), Lagos Quintana et al, Science 294, 853-857 (2001), and Lagos-Quintana et al, RNA, 9, 175-179 (2003), which are incorporated by reference. Multiple microRNAs can also be incorporated into the precursor molecule.

The target sites in the mRNA may be in the 5' UTR, the 3' UTR or in the coding region. Interestingly, multiple miRNAs may regulate the same mRNA target by recognizing the same or multiple sites. The miRNA may specify cleavage of the mRNA if the mRNA has a certain degree of complementarity to the miRNA. When a miRNA guides cleavage, the cut may be between the nucleotides pairing to residues 10 and 11 of the miRNA. Alternatively, the miRNA may repress translation if the miRNA does not have the requisite degree of complementarity to the miRNA. Translational repression may be more prevalent in animals since animals may have a lower degree of complementarity between the miRNA and binding site.

There is practically no limitation as to the nature of the miRNAs that can be incorporated in the polynucleotides of the invention. Thus, any miRNA previously known and described in the miRBase Sequence Database (described in by Griffiths-Jones S, et al., 2006, Nucleic Acids Res., 34 (Database Issue):D140-D144) and accessible at http://microrna.sanger.ac.uk/sequences/ can be incorporated in the polyucleotides of the invention and includes miRNA isolated from organisms such as *Anopheles gambiae, Ateles geoffroyi, Apis mellifera, Arabidopsis thaliana, Bombyx mori, Brassica napus, Bos Taurus, Caenorhabditis briggsae, Caenorhabditis elegans, Canis familiaris, Ciona intestinalis, Chlamydomonas reinhardtii, Ciona savignyi, Drosophila ananassae, Drosophila erecta, Drosophila grimshawi, Drosophila melanogaster, Drosophila mojavensis, Drosophila persimilis, Drosophila pseudoobscura, Drosophila sechellia, Drosophila simulans, Danio rerio, Drosophila virilis, Drosophila willistoni, Drosophila yakuta,* Epstein Barr virus, *Fugu rubripes, Gallus gallus, Gorilla gorilla, Gossypium hirsutum, Glycine max, Human* cytomegalovirus, HIV-1, *Homo sapiens,* Kaposi sarcoma-associated herpesvirus, *Lemur catta, Lagothrix lagotricha,* Mouse cytomegalovirus, *Monodelphis domestica,* Mareks disease virus type 1 and type 2, Mouse gammaherpesvirus, *Macaca mulatta, Mus musculus, Macaca nemestrina, Medicago truncatula, Ornithorhynchus anatinus, Oikopleura dioica, Oryza sativa, Pygathrix bieti, Pan paniscus, Physcomitrella patens, Pongo pygmaeus, Pinus taeda, Populus trichocarpa, Pan troglodytes, Rhesus lymphocryptovirus, Rattus norvegicus, Sorghum bicolor, Saguinus labiatus, Solanum lycopersicum, Schmidtea mediterranea, Selaginella moellendorffii, Saccharum officinarum, Sus scrofa, Symphalangus syndactylus, Triticum aestivum, Tribolium castaneum, Tetraodon nigroviridis, Vitis vinifera, Xenopus tropicales, Zea mays,*

miRNAs can be identified using a combination of the expression and biogenesis criteria defined by Ambros et al. (RNA, 2003, 9:277-279) as follows:
- A: Detection of distinct 22 nt RNA transcript in size-fractionated RNA sample,
- B: Identification of the 22 nt sequence in a cDNA libray made from size fractionated RNA.
- C: Prediction of a potential fold-back precursor structure containing the 22 nt miRNA sequence within one arm of the hairpin.
- D: Phylogenetic conservation of the 22 nt mRNA sequence and its precited fold-back precursor secondary structure.
- E: Detection of increased precursor accumulation in organisms with reduced Dicer function.

Preferred miRNA for use in the polynucleotides of the present invention include, without limitation, miR-22, miR-26a-1, miR-26a-2, miR- 29b-2, miR-29c, miR-30e, miR-30c-1, miR-146a, miR-150, let-7a-1, let-7f-1, let-7d, miR- 100, let-7a-2, miR-125b-1, let-7a-3, let-7b, miR-99a, let-7c, miR-125b-2, miR-99b, let-7e, miR-125a, let-7f-2, miR-98, let-7g, let-7i, miR-26b, miR-30c, miR-34a, miR-150, miR- 195/497, miR-15a/16-1.

The polynucleotide of the invention maybe a single-stranded or a double-stranded polynucleotide. In a preferred embodiment, the polynucleotide a double stranded DNA and wherein the sequence encoding the sense strand forms a duplex with the sequence encoding the antisense strand. The polynucleotide would be comprised by a double stranded promoter region, a double stranded region comprising the sense and antisense strand of the miRNA and a double stranded second promoter region showing promoting the transcription of the miRNA antisense strand and wherein both promoters act convergently.

The sequences encoding the sense strand and/or the sequence encoding the antisense strand may be followed by transcriptional termination signals. Non-limiting examples of transcriptional terminators suitable for use in the present invention include the SV40 terminator and the growth hormone gene terminator. Suitable transcriptional termination signals comprise a sequence of ca. 4 adenine residues. In those cases
wherein the polynucleotide of the invention is a double stranded polynucleotide, the transcriptional termination signal contains a 4 thymidine residues region in the complementary strand. The polyadenine and polythymidine regions are preceded by a GC-rich region having palindrome symmetry. Transcription proceeds normally along the polyadenine region and terminates before the GC-rich region, thus resulting in that the transcript ends by one or more uridine residues.

The polynucleotides of the invention can be placed into suitable vectors for propagation and for further study. Practically any vector known in the art may be suitable for carrying out the invention provided that suitable promoter regions and endonuclease target sites are inserted into the vectors. Thus, suitable vectors for the insertion of the polynucleotides of the invention include expression vectors in prokaryotes such as pUC18, pUC19, Bluescript and their derivatives, mp18, mp19, pBR322, pMB9, CoIE1, pCR1, RP4, phages and vectors "shuttle" such as pSA3 and pAT28, expression vectors in yeast such as vectors of the type of plasmids of 2 microns, integration plasmids, YEP vectors, centromeric plasmids and similar, expression vectors in insect cells such as the vectors of the pAC series and pVL series, expression vectors in plants such as vectors of the series pIBI, pEarleyGate, pAVA, pCAMBIA, pGSA, pGWB, pMDC, pMY, pORE and similar and expression vectors in eukaryotic cells either based on viral vectors (adenovirus, adenovirus-associated virus, as well as retrovirus and, in particular, lentivirus) as well as non-viral vectors such as pSilencer 4.1-CMV (Ambion), pcDNA3, pcDNA3.1/hyg pHCMV/Zeo, pCR3.1, pEF1/His, pIND/GS, pRc/HCMV2, pSV40/Zeo2, pTRACER-HCMV, pUB6/V5-His, pVAX1, pZeoSV2, pCI, pSVL and pKSV-10, pBPV-1, pML2d and pTDT1. Vectors particularly appropriate for the insertion of said polynucleotides are expression vectors in superior eukaryotic cells, either based on viral vectors such as, without limitation, Moloney murine leukemia virus; Murine stem cell virus, Harvey murine sarcoma virus; murine mammary tumor virus; Rous sarcoma virus; adenovirus; adeno-associated virus; SV40-type viruses; polyoma viruses; Epstein- Barr viruses; papilloma viruses; herpes viruses; vaccinia viruses; polio viruses; lentiviruses; and RNA viruses such as any retrovirus. Viral vectors are generally based on non-cytopathic eukaryotic viruses in which non-essential genes have been replaced with the nucleic acid sequence of interest. In a preferred embodiment, said expression vector is suitable for transfecting or infecting mammal cells *ex vivo,* particularly human cells.

In a preferred embodiment, the vectors are lentiviral vectors. Recombinant lentiviral vectors refer to vectors with sufficient genetic information to allow packaging of an RNA genome, in the presence of packaging components, into a viral particle capable of infecting and transducing a target cell. A recombinant lentiviral vector is incapable of independent replication to produce infectious retroviral particles within the final target cell. Preferably the recombinant lentiviral vector has a minimal viral genome. Moreover, the lentiviral-based systems are capable of integrating the proviral DNA in the genome of the cell. This feature makes possible to isolate the DNA fragment corresponding to the miRNA cloned into the lentivirus. Genomic DNA isolated can be amplified with the H1 (5' CTGGGAAATCACCATAAACGTGAA 3') (SEQ ID NO:7) and U6 (5' GCTTACCGTAACTTGAAAGTATTTCG 3') (SEQ ID NO:8) primers. PCR products can be subcloned and sequenced.

Lentiviral vectors include primate lentiviral vectors such as HIV vectors (for example, HIV-I and HIV-2 vectors) and SIV vectors, and non-primate lentiviral vectors. Primate lentiviral vectors have a number of disadvantages which may limit their therapeutic application to certain diseases Therefore, in a particularly preferred embodiment, the lentiviral vector will be a non-primate lentiviral vector, such as EIAV, FIV, BIV, CAEV or MVV. In a still more preferred embodiment, the lentiviral vectors derive from the feline immunodeficiency virus.

The vectors of the invention may comprise a reporter or marker gene that allows the identification of cells that have incorporated the vector after having been put in contact with the vector. Reporter genes suitable for use in the present invention include LacZ, luciferase, thymidine kinase, GFP, DsRed and the like.

### Library of the invention

In another aspect, the invention provides a library comprising a plurality of the polynucleotides of the invention or of the vectors of the invention.

As used herein, the term "library" refers to a collection of nucleic acid molecules (circular or linear). In one embodiment, a library may comprise a plurality of nucleic acid molecules (e.g., two, three, four, five, seven, ten, twelve, fifteen, twenty, thirty, fifty, one hundred, two hundred, five hundred one thousand, five thousand, or more), which may or may not be from a common source organism, organ, tissue, or cell. In another embodiment, a library is representative of all or a portion or a significant portion of the nucleic acid content of an organism (a "miRNA" library) in a cell, tissue, organ or organism. A library according to the invention can comprise at least one representative for substantially all the miRNAs present in a organism, organ, tissue or cell or defined percentages of it, e.g., at least 20, 25, 30, 35, 40, 45, 50, 55, 60, 65, 70, 75, 80, 85, 90, 95, 97, 99% or more (and values in between). A library may also comprise nucleic acid molecules having random sequences made by de novo synthesis, mutagenesis of one or more nucleic acid molecules, and the like. Such libraries may or may not be contained in one or more vectors (e.g., two, three, four, five, seven, ten, twelve, fifteen, twenty, thirty, fifty, etc.).

The polynucleotides of the invention can be introduced into a cell, thus resulting in the expression of the miRNA in said host cell. Thus, in another embodiment, the invention relates to a host cell comprising a polynucleotide of the invention, a vector of the invention or a library of the invention.

The host cells of the invention can also be used in a method for expressing a miRNA in a host cell. Thus, in another embodiment, the invention relates to a method for expressing a miRNA in a host cell comprising contacting the cell with a polynucleotide of the invention, with a vector of the invention or with a library of the invention under conditions adequate for entry of the polynucleotide or the vector in the cell and for the expression of said polynucleotide in the cell.

The polynucleotide, vector or library of the invention can be introduced in the cells using any of the transfection methods known by the persons skilled in the art (see sections 9.1 to 9.5 in Ausubel, F.M. et al., Current Protocols in Molecular Biology, John Wiley & Sons Inc; ringbou edition, 2003). The cells can be transfected by means of precipitating DNA with calcium phosphate, DEAE-dextran, polybrene, electroporation, microinjection, liposome-mediated fusion, lipofection, infection by retrovirus and biolistic transfection.

In the particular case that the polynucleotides of the invention form a library, the library can be expressed in a host cell arrays. A host cell array can be produced according to any suitable method. For example, host cells can be distributed into multi-well plates, and then transformed with a polynucleotide according to conventional methods. In addition, microarrays of cells expressing polynucleotides can be created, e.g., according to Ziauddin and Sabatini (Nature, 411, 107-110, 3 May 2001), where cDNAs are printed on printed onto a glass slide, exposed to a transfection reagent (e.g., a lipid), and then covered with adherent mammalian cells.

For many purposes, it may be advantageous to utilize adherent cells that can be cultured on a solid support and which remain on the support when processed to determine the presence or absence of a specific-binding partner. Adherent cells are well-known, and the surfaces can be treated accordingly to provide appropriate surfaces for adherence to take place. For instance, surfaces can be treated with poly-anionic amino acids (e.g., polylysine), collagen, integrins, gelatin, and other molecules and substances which facilitate cell adhesion.

Any type of host cell can be utilized without limitation. These include, e.g., bacteria, yeast, eukaryotic, animal, mammalian (e.g., COS, CMV1, BHK, CHO, HeLa, LTK, NIH 3T3, 293), plant, etc., cells. When a polynucleotide is "expressible," it is operably linked to an expression control sequence (e.g., promoter) in such a way that facilitates transcription and translation of the polynucleotide's coding sequence.

### Method for the preparation of a polynucleotide of the invention

The authors of the present invention have also developed a method for the preparation of a vector for expressing a miRNA (hereinafter, first method of the invention). Thus, in another aspect, the invention relates to a method for the preparation of a vector for expressing a miRNA comprising the steps of
(i) contacting a miRNA molecule with a 5' adapter and a 3' adapter
   wherein each adapter comprises a known sequence, wherein said contacting step is carried out under conditions adequate for promoting ligation of the adapters to the 5' and 3' ends of the RNA and for preventing circularization of the RNA,
(ii) reverse transcribing the 5'-adapter-RNA-3'adapter molecule obtained in step (i) using a primer which sequence is substantially complementary to the sequence of the 3' adapter,
(iii) producing a double stranded polynucleotide using as template the cDNA obtained in step (ii) and
(iv) inserting the polynucleotide obtained in step (iii) in a suitable vector
wherein convergent promoter sequences are present either within the known sequences of the 5' and 3' adapters or 5' or 3 with respect of the site of the vector wherein the polynucleotide is inserted in step (iv).

Step (i) of the first method of the invention involves contacting a miRNA molecule with a pair of adapters wherein each adapter comprises a known region. The known region present in the 5' and 3' adapter may be any region which can then be used as template for the design of primers suitable for PCR amplification of the miRNA derived sequences. Alternatively, the known regions in the 5' and 3' adapter may comprise target sites for restriction endonucleasas that may be the same or different. These sites will allow the generation of cohesive ends that may be used for ligation into a vector previously treated with the same enzymes or with enzymes generating compatible cohesive ends with the ends present in the miRNA derived insert. Alternatively, the regions of known sequences in the adapter contain sequences corresponding to promoters, wherein said promoters are acting convergently, i.e. the 5' adapter comprises a promoter sequence that promotes transcription of downstream sequences and the 3' adapter comprises a promoter sequence that promotes transcription of upstream sequences. It is to be understood that the regions of known sequences may contain one or more of the elements mentioned before. Thus, it is possible that the regions contain both promoter sequences and recognitions sites for restriction endonucleasas. In this case, the target sites for the restriction endonucleases are located 5' with respect to the promoter regions. Although it is possible to combine in the region of known sequence both a promoter sequence and a second sequence that can be used as template for the design of PCR amplification primers, the promoter regions may serve themselves as amplification target sites.

The contacting step (i) is carried out under conditions adequate for promoting ligation of the adapters to the 5'and 3' ends of the RNA while preventing circularization of the RNA. The skilled person will appreciate that any method known in the art for ligating nucleic acid sequences to a RNA can be used in the context of the present invention. In particular, step (i) can be carried out using either the basic protocol or the alternate protocol as described by Ausubel et al. (Ausubel et al., 2003, Current Protocols in Molecular Biology, 26.4.1-26.4.18).

In the basic protocol, the miRNA molecule is first dephosphorylated, preferably with alkaline phosphatase, to prevent circularization of the RNA. A 5' phosphorylated oligonucleotide with a blocked 3-hydroxyl group is attached to the small RNAs. The ligation product is separated from excess 3'-adapter and is subsequently 5'-phosphorylated and subjected to 5'-adapter ligation.

In a preferred embodiment, ligation of the adapter molecules is carried out using an alternate protocol which comprises the steps of contacting the miRNA with a 3' adapter which carries a reactive group at the 5' terminus (preferably an adenyl group) and a protecting group at the 3' terminus (preferably a dideoxy C group). The ligation is carried out in the absence of ATP, which prevents recircularization of the ARN during T4 RNA ligation and thus, bypasses the need for the dephosphorylation of the miRNA. The ligation step is carried out in the presence of a RNA ligase (preferably T4 RNA ligase). The 5' adapter is then ligated to the in the presence of ATP and a RNA ligase (T4 RNA ligase). In a preferred embodiment, the 5' adapter is a hybrid DNA/RNA sequence. In a still more preferred embodiment, the RNA part of the hybrid 5' adapter corresponds to the 3' region of the adapter.

The 5'-adapter-RNA-3'adapter molecule obtained in step (i) may be purified by conventional means. Preferably, a size fractionation is carried out in order to eliminate concatemers formed during step (i).

Step (ii) comprises the reverse transcription of the 5'-adapter-RNA-3'adapter molecule obtained in step (i) using a primer which sequence is substantially complementary to the sequence of the 3' adapter. Reverse transcription is carried out using methods known to the skilled person and can be carried out isothermally as well as using thermostable RNA polymerases in the presence of a RNA-dependent DNA polymerase including, without limitation, AMV, Cloned AMV, MMLV, SuperscriptII, ReverTraAce, Tth reverse transcriptase, hepatitis B reverse transcriptase, cauliflower mosaic virus reverse transcriptase, bacterial reverse transcriptase and Thermoscript. An enzyme or reverse transcriptase that is used for nucleic acid synthesis may be purified from a virus or bacterium, or it may be prepared via gene recombination. Alternatively, such enzyme may be subjected to modification such as fragmentation or amino acid substitution. The primer used in the reverse transcription reaction is substantially complementary to the sequence of the 3' adapter. The term "substantially complementary", as used herein, refers to a nucleic acid that will selectively hybridize to one another under particular conditions, or to an oligonucleotide analogue and a nucleic acid molecule that will selectively hybridize to one another under particular conditions, but may contain mismatched bases at one or more positions.

The enzymes for use in the invention include those that have reduced, substantially reduced or eliminated RNase H activity. By an enzyme "substantially reduced in RNase H activity" is meant that the enzyme has less than about 20%, more preferably less than about 15%, 10% or 5%, and most preferably less than about 2%, of the RNase H activity of the corresponding wild-type or RNase H+ enzyme such as wild-type Moloney Murine Leukemia Virus (M-MLV), Avian Myeloblastosis Virus (AMV) or Rous Sarcoma Virus (RSV) reverse transcriptases. The RNase H activity of any enzyme may be determined by a variety of assays, such as those described, for example, in U.S. Patent No. 5,244,797 , in Kotewicz, M.L., et al., Nucl. Acids Res. 16:265 (1988) and in Gerard, G.F., et al., FOCUS 14(5):91 (1992). Particularly preferred polypeptides for use in the invention include, but are not limited to, M-MLV reverse transcriptase, RSV reverse transcriptase, AMV reverse transcriptase, RAV (rous-associated virus) reverse transcriptase, MAV (myeloblastosis-associated virus) reverse transcriptase and HIV reverse transcriptase. (See U.S. Patent No. 5,244,797 and WO 98/47912). It will be understood by one of ordinary skill, however, that any enzyme capable of producing a DNA molecule from a ribonucleic acid molecule (i.e., having reverse transcriptase activity) may be equivalently used in the compositions, methods and kits of the invention.

The single stranded cDNA obtained in step (ii) can be treated so as to obtain a double-stranded DNA using any method known in the art. Preferably, the conversion of the single stranded cDNA to the double stranded DNA is carried out using in vitro amplification technologies such as Polymerase Chain Reaction (PCR), Ligase Chain Reaction (LCR), Nucleic Acids Sequence Based Amplification (NASBA), Strand Displacement Amplification (SDA), Transcription Mediated Amplification (TMA), Branched DNA technology (bDNA), linker-aided DNA amplification (LADA), Q-beta replicase amplification (Q-beta), loop-mediated isothermal amplification (LAMP) and Rolling Circle Amplification Technology (RCAT), or other in vitro enzymatic amplification technologies. The amplification step is carried out using primers corresponding to the sequences of the adapter regions. The resulting double-stranded DNA can be purified using a purification column, electromagnetic beads to which the primer is attached, or by electrophoresis through a polyacrylamide gel.

Preferably, the primers used in the PCR reaction are modified with a first member of a binding pair so that the excess of primer not incorporated into the double stranded DNA can be purified using the second member of the binding pair when coupled to an affinity matrix. Suitable binding pairs for the purification of the double-stranded DNA include, without limitation, hapten or antigen/antibody (e.g. digoxin and anti-digoxin antibodies), biotin or biotin analogues (e.g. aminobiotin, iminobiotin or desthiobiotin)/avidin or streptavidin, sugar/lectin, enzyme and cofactor, folic acid/folate, double stranded oligonucleotides that selectively bind to proteins/, transcription factors, nucleic acid or nucleic acid analogue/complementary nucleic acid, receptor/ligand (e.g., steroid hormone receptor/steroid hormone).

The resulting double stranded DNA can then be inserted into a vector of choice using methods known in the art. In a preferred embodiment, the primers used during the PCR-amplification step contain within their 5' regions target sites for restriction endonucleases which generate compatible ends with those present in the vector of choice. The endonuclease target sites allow the generation of cohesive ends that can be used for cloning the polynucleotides in appropriate vectors. Preferably, the endonuclease target sites do not show any homology to sites within native miRNA present in the human genome. Adequate restriction sites can be identified using the Blast-n algorithm with the cleavage site or selecting restriction endonuclease whose target sites comprise 7 or 8 nucleotides, thus appearing in the human genome only sporadically. Non-limiting examples of endonuclease target sites that can be used in the adapters for use in the method of the invention include BvbII and the like. Non-limiting examples of enzymes having recognitions sites comprising 7, 8 or more nucleotides are described in the restriction endonuclease database REBASE (Roberts RJ, Macelis D. (2001) REBASE--restriction enzymes and methylases. Nucleic Acids Res. 2001 Jan 1; 29: 268-9 y Roberts, R. J. and Macelis, D. (1999) Nucleic Acids Res., 27,312-313) and incluye, without limitation, I-Ceu I, PI-Sce I, TevII, Bmol, Dmol, Fsel, Pad, Pmel, Psrl, Bcgl, BgII, Bsabl, BstXI, Drdl, EcoNI, Fsel, MaM I, MsI 19 Mwo I, Psha I, Sfi I, Swa I, Xcm I, Xmn I and the like, as well as the so-called meganucleasas (described for instance in international patent application WO2003078619).

In a preferred embodiment, the invention contemplates the use of restriction endonucleases sites for enzymes that cut outside said recognition sites. In this way, it is possible to obtain double stranded polynucleotides that do not contain any nucleotides of the target site. non-limiting examples of said enzymes incluye AlwI, BbsI, BbvI, BbvII, BceA1, BciV1, BfuAl, Brnr1, BpmI, BpuEl, BsaI, BseR1, Bsgl, BsmAl, BsmB1, BsmF1, BspM1, EarI, EciI, FauI, FokI, HgaI, HphI, NboII, MlyI, MnlI, PleI, SapI y SfaI. In a preferred embodiment, the restriction endonuclease target site consists of the sequence GAAGACN₂/N₆, corresponding to the recognition site for the BbvII restriction endonuclease.

The insertion of the polynucleotides of the invention into the vectors having cohesive ends is carried out using any of the methods known in the art for preparing hybrid DNA molecules from two or more fragments and involve preferably the use of DNA ligases such as T4 or E.coli DNA ligase *E.coli* under the conditions recommended by the supplier. Exemplary methods for ligating DNA molecules are described in retail under sections 3.14, 3.16 y 3.17 of Ausubel,F.M. et al., (Current Protocols in Molecular Biology, John Wiley and Sons, eds. 2003).

Suitable vectors for inserting the polynucleotides obtained according to the method have been described previously. In a preferred embodiment, the vector may comprise promoter regions flanking the restriction endonuclease target site wherein the insert is to be placed. In this embodiment, the adapters used in step (i) are preferably devoid of promoter regions themselves since these will be provided by the vector. In a preferred embodiment, the vector is a lentiviral vector.

### Method for the preparation of a miRNA library of the invention

The authors of the present invention have also developed a method for the preparation of a miRNA library based on the polynucleotides of the invention (hereinafter the second method of the invention). Thus, in another aspect, the invention relates to a method of preparing a library for expressing a miRNA comprising the steps of
(i) contacting a RNA preparation comprising at least one miRNA molecule having about 19-25 nucleotides in length with a 5' adapter and a 3' adapter of known sequences, wherein said contacting step is carried out under conditions adequate for promoting ligation of the adapters to the 5' and 3' ends of the RNA and for preventing circularization of the RNA,
(ii) reverse transcribing the 5'-adapter-RNA-3'adapter molecules obtained in step (i) using a primer which sequence is substantially complementary to the sequence of the 3' adapter,
(iii) producing double stranded polynucleotides using as template the cDNAs obtained in step (ii) and
(iv) inserting the polynucleotides obtained in step (iii) in a suitable vector
wherein convergent promoter sequences are present either within the known sequences of the 5' and 3' adapters or 5' or 3 with respect of the site of the vector wherein the polynucleotide is inserted in step (iv).

The term, as used herein, is understood as any sample comprising at least one miRNA, more preferably, a plurality of miRNA molecules having 19-25 nucleotides. In a preferred embodiment, the RNA preparation has been obtained from a cell and comprises a representative population of all the miRNA expressed in a cell as well as of any other endogenous small RNA present in the cell. The RNA preparation is prepared using any method known in the art for isolating RNA including treatment of the cells sequentially with 4 M guanidinium salt, sodium acetate (pH 4), phenol, and chloroform/isoamyl alcohol followed by centrifugation of the samples and RNA precipitation from the upper layer by the addition of alcohol (P. Chomczynski, Anal. Biochem., 162, 156-159 (1987)); contacting the cell lysate with which a stable mixture of phenol and guanidinium salt at an acidic pH and, after phase separation with chloroform, recovering the RNA in the aqueous phase by precipitation with an alcohol (US 4843155); binding on glass or other solid phases (US5234809); binding to diatomaceous earth or silica particles of nucleic acids present in lysates obtained by exposure to strong (5 M) solutions of guanidinium thiocyanate in Tris HCl (pH 8.0), containing EDTA and the surfactant Triton X-100; precipitating RNA and DNA from cell lysates in the presence of singly charged monomeric cationic surfactants (US5010183 and US5985572). In another embodiment, the RNA is isolated using methods specifically designed for the isolation of low molecular weight RNA such as the method described in WO2005054466.

Preferably, the RNA is isolated from cells harvested during their exponential growth phase. Some tissues, like pancreas or small intestine, are rich in nucleases and intrinsically difficult to handle. It is recommended that the quality of the RNA preparation be carefully examined before making the decision to start a lengthy cloning protocol. High-quality total RNA shows defined 18S and 28S rRNA bands by agarose gel electrophoresis.

The yield of RNA obtained can be determined using any method known in the art. By way of example, the yield of RNA can be determined by Nuclease Protection Assays (NPAs), including both ribonuclease protection assays (RPAs) and S1 nuclease assays, denaturing agarose gel electrophoresis followed by blotting the contents of these gels onto hybridization membranes and probing with radioactive oligonucleotide (RNA or DNA-based) probes and UV Absorbance wherein the concentration and purity of RNA can be determined by diluting an aliquot of the preparation (usually a 1: 50 to 1 : 100 dilution) in TE (10 mM Tris-HCl pH 8,1 mM EDTA) or water, and reading the absorbance in a spectrophotometer at 260 nm and 280 nm. Alternatively, a more modern RNA LabChip technique may be used for analyzing the quality of the total RNA. LabChip technologies (e.g., Agilent 2100 Bioanalyzer) were developed for quality control of total RNA to be used for gene profile analysis on DNA arrays.

Another difficulty is the handling of extremely low amounts of RNA products making it necessary to use surface-treated, siliconized reaction tubes. Extreme care is needed to avoid nuclease contamination throughout the protocol.

Finally, although it is feasible to analyze the cloned sequences by hand, it is recommended that the assistance of an experienced bioinformatics specialist be sought to develop adequate analysis and archiving tools. The small RNA fractions often contain substantial amounts of degradation products of other abundant noncoding RNAs such as rRNAs, tRNAs, snRNAs, and snoRNAs, as well as messenger RNAs. A guideline to the annotation of newly identified miRNA genes has been published (Ambros et al., 2003) and a repository for miRNAs can be found at http://www.sanger.ac.uk/Software/Rfam/index.shtml.
Once a population of miRNA is available, the ligation of the 5' and 3' adapters (step (i)) and the generation of a cDNA from the 5' adapter-miRNA-3' adapter (step (ii)) is carried out essentially as described in the previous method of the invention. If desired, the library obtained using the first of the invention can be treated essentially as described in the previous method so as to obtain double-stranded DNA having cohesive ends which can then be inserted into a suitable vector having compatible cohesive ends. Preferably, the vector used for this purpose is a lentiviral vector as previously described.

### Method for the identification of miRNAs capable of interfering in a cellular process

The miRNA libraries of the present invention can be screened in order to identify miRNAs capable of interfering in a cellular process. Thus, in another aspect, the invention relates to a method (hereinafter the third method of the invention) for the identification of miRNAs capable of interfering in a cellular process comprising
(i) contacting a miRNA library of the invention with a cell population under conditions adequate for promoting the entry of the library polynucleotides in the cell,
(ii) maintaining the cells under conditions adequate for promoting expression of the library-encoded miRNAs,
(iii) selecting those cells showing an alteration in the cellular process under study and
(iv) recovering the library polynucleotides from the cell.

In step (i), the miRNA library is introduced in the cells being studied using any of the transfection methods known by the persons skilled in the art (see sections 9.1 to 9.5 in Ausubel, F.M. et al., Current Protocols in Molecular Biology, John Wiley & Sons Inc, 2003).

The cell under study may be derived from or contained in any organism (e.g., plant, animal, protozoan, virus, bacterium, or fungus). The cell wherein the library may be inserted may be derived from or contained in any organism. The organism may be a plant, animal, protozoan, bacterium, virus, or fungus. The plant may be a monocot, dicot or gymnosperm; the animal may be a vertebrate or invertebrate. Preferred microbes are those used in agriculture or by industry, and those that are pathogenic for plants or animals. Fungi include organisms in both the mold and yeast morphologies.

Plants include arabidopsis; field crops (e.g., alfalfa, barley, bean, corn, cotton, flax, pea, rape, rice, rye, safflower, sorghum, soybean, sunflower, tobacco, and wheat); vegetable crops (e.g., asparagus, beet, broccoli, cabbage, carrot, cauliflower, celery, cucumber, eggplant, lettuce, onion, pepper, potato, pumpkin, radish, spinach, squash, taro, tomato, and zucchini); fruit and nut crops (e.g., almond, apple, apricot, banana, blackberry, blueberry, cacao, cherry, coconut, cranberry, date, fajoa, filbert, grape, grapefruit, guava, kiwi, lemon, lime, mango, melon, nectarine, orange, papaya, passion fruit, peach, peanut, pear, pineapple, pistachio, plum, raspberry, strawberry, tangerine, walnut, and watermelon); and ornamentals (e.g., alder, ash, aspen, azalea, birch, boxwood, camellia, carnation, chrysanthemum, elm, fir, ivy, jasmine, juniper, oak, palm, poplar, pine, redwood, rhododendron, rose, and rubber).

Examples of vertebrate animals include fish, mammal, cattle, goat, pig, sheep, rodent, hamster, mouse, rat, primate, and human. Invertebrate animals include nematodes, other worms, drosophila, and other insects. Representative generae of nematodes include those that infect animals (e.g., Ancylostoma, Ascaridia, Ascaris, Bunostomum, Caenorhabditis, Capillaria, Chabertia, Cooperia, Dictyocaulus, Haernonchus, Heterakis, Nematodirus, Oesophagostomum, Ostertagia, Oxyuris, Parascaris, Strongylus, Toxascaris, Trichuris, Trichostrongylus, Tflichonema, Toxocara, Uncinaria) and those that infect plants (e.g., Bursaphalenchus, Criconerriella, Diiylenchus, Ditylenchus, Globodera, Helicotylenchus, Heterodera, Longidorus, Melodoigyne, Nacobbus, Paratylenchus, Pratylenchus, Radopholus, Rotelynchus, Tylenchus, and Xiphinerna). Representative orders of insects include Coleoptera, Diptera, Lepidoptera, and Homoptera.

The cell may be from the germ line or somatic, totipotent or pluripotent, dividing or non-dividing, parenchyma or epithelium, immortalized or transformed, or the like. The cell may be a stem cell or a differentiated cell. Cell types that are differentiated include adipocytes, fibroblasts, myocytes, cardiomyocytes, endothelium, neurons, glia, blood cells, megakaryocytes, lymphocytes, macrophages, neutrophils, eosinophils, basophils, mast cells, leukocytes, granulocytes, keratinocytes, chondrocytes, osteoblasts, osteoclasts, hepatocytes, and cells of the endocrine or exocrine glands.

The cells can particularly be transfected by means of precipitating DNA with calcium phosphate, DEAE-dextran, polybrene, electroporation, microinjection, liposome-mediated fusion, lipofection, infection by retrovirus and biolistic transfection.

The polynucleotides inserted in the cell can be maintained transitorily or stably. For the purpose of obtaining stable expression of the polynucleotides of the invention, it is necessary to include in the transfection a gene encoding resistance to a certain antibiotic, such that those cell lines which have incorporated the DNA in the genome of those cell lines in which the DNA is in an extrachromosomal position can be screened. The gene which allows screening the cells can be provided forming part of the same vector containing the construct object of the invention or, alternatively, it can be provided separately by means of cotransfecting a second plasmid containing said resistance gene. In the latter case, the plasmid containing the DNA construct is provided to the transfection mixture in a molar excess with respect to the resistance gene, such that for each resistance gene integration event there is a high probability of integration of the gene containing the promoter being studied. The plasmid containing the DNA construct is preferably provided in an excess of at least 5 times with respect to the vector containing the resistance reporter.

Suitable resistance markers for screening cell lines which have integrated the construct in the genome include positive selection markers such as, for example, the neomycin resistance gene, conferring resistance to the aminoglycoside G418, the hygromycin phosphotransferase gene conferring resistance to hygromycin, the ODC gene conferring resistance to the ornithine decarboxylase inhibitor (2-(difluoromethyl)-DL-ornithine (DFMO)), the dihydrofolate reductase gene conferring resistance to methotrexate, the puromycin-N-acetyl transferase gene conferring resistance to puromycin, the ble gene conferring resistance to zeocin, the adenosine deaminase gene conferring resistance to 9-beta-D-xylofuranosyl adenine, the cytosine deaminase gene allowing the cells to grow in the presence of N-(phosphonacetyl)-L-aspartate, thymidine kinase allowing the cells to grow in the presence of aminopterine, the Xanthine-guanine phosphoribosyltransferase gene allowing the cells to grow in the presence of xanthine and in the absence of guanine, the *E.coli* trpB gene allowing the cells to grow in the presence of indole instead of tryptophan, the *E.coli* hisD gene allowing the cells to use histidinol instead of histidine. The selection gene is incorporated in a plasmid which can additionally include a suitable promoter for the expression of said gene in eukaryotic cells, (for example the CMV or SV40 promoters), an optimized translation initiation site (for example a site following the so-called Kozak rules or an IRES), a polyadenylation site such as for example the SV40 or phosphoglycerate kinase polyadenylation site, introns such as for example the beta-globulin gene intron.

The screening process of cells containing the DNA construct of interest stably integrated in the genome is carried out by means of a conventional screening process (see for example Ausubel, F.M. et al., Current Protocols in Molecular Biology (1997) 9.5.1-9.5.19). To that end, the cells are transfected with a vector or mixture of vectors and, after a recovery period, they are allowed to grow in a selective medium (either a medium containing the antibiotic against which the reporter confers resistance or a minimum medium containing the antimetabolite against which the reporter confers resistance). The cell colonies growing in selective medium are isolated and allowed to grow again in selective medium. Once cells have been obtained which can grow during repeated proliferation cycles in the presence of the selection marker, it may be convenient to eliminate said marker from the cells, particularly if the cells are to be transfected with another selection marker. To that end, recombinases may be used, particularly the Cre/Lox system. It is alternatively possible to amplify the number of copies of the selection marker, which results in a simultaneous amplification of the number of copies of the gene of interest, with the subsequent increase in its expression.

To that end, the cells are grown in the presence of progressively greater concentrations of selection agent, which results in a screening of the cells which have undergone an amplification of the genes conferring resistance to such agent and normally of the adjacent or intermediate regions. DHFR is preferably used as a selection marker and the screening of cell lines in which there is an amplification of said gene is carried out in the presence of methotrexate.

A cell is normally considered to stably express a marker when the expression of said marker does not decrease with successive proliferation cycles, independently of the presence of selection agent in the culture medium.

Once present in a host cell, the polynucleotide can be extra-chromosomal or integrated into a chromosome(s) of the host cell. The polynucleotide can be stably or transiently expressed, depending on the desired purpose.

In step (ii), the cells are maintained under conditions suitable for expressing the polynucleotide introduced in the cell in step (i). Expression of the polynucleotide can be carried out by endogenous RNA polymerase of the cell. Suitable conditions include any culture conditions which are adequate for achieving production of the polypeptide by the host cell, including effective temperatures, pH, medium, additives to the media in which the host cell is cultured (e.g., additives which amplify or induce expression such as butyrate, or methotrexate if the coding polynucleotide is adjacent to a DHFR gene), cycloheximide, cell densities, culture dishes, etc. If desired, the cells can be tested for expression of the polynucleotides using methods widely known to the skilled person.

In step (iii) of the method, cells showing a particular phenotype are selected. The term "phenotype" refers to any process occurring in or between one or more host cells. Thus, phenotypes that can be used tested using in step (iii) include, but are not limited to, signal transduction, growth, proliferation development, differentiation, metabolism, disease resistance, cell division, secretion, transcription, translation, splicing, cell-cell communication, endocytosis, exocytosis, antigen presentation, cell death, and the like.

The phenotype may be an abnormal (mutant or pathological) process. For example, the methods of the invention may be used to determine an effect on a pathological biological process, such as modifying or abolishing the pathological process.

The term "showing a phenotype" refers to any measurable qualitative or quantitative effect on a biological process in the one or more host cells or their progeny. The effect may be measured, for example, as a morphological, biochemical, physiological, molecular, cellular, behavioural effect on the level of single cells, collection of cells, tissues, organs, or whole organisms. Depending on the assay, a phenotypic effect may be if there is an inhibition which is greater than 10%, 33%, 50%, 90%, 95% or 99% as compared to a cell not treated according to the present invention. Lower doses of injected material and longer times after administration of dsRNA may result in inhibition in a smaller fraction of cells (e.g., at least 10%, 20%, 50%, 75%, 90%, or 95% of targeted cells).

The method of the invention also allows the identification of miRNAs capable of inducing phenotypic alterations in a complete organism, In this case, it is required to generate animal models capable of expressing the polynucleotide of the invention, either constitutively or inducible. Methods for the generation of transgenic mammals, fish, birds and insects are widely known to the skilled person. Said methods may involve contacting retroviral particles, in particular, lentiviral particles carrying the constructs comprising the polynucleotides of the invention with cells (oocytes, zygotes or early-stage embryos), transfecting embryonic stem cells followed by the incorporation of said cells in an embryo or generating oocytes or sperm cells by in vitro differentiation of embryonic stem cell carrying the polynucleotides of the invention integrated in their genome.

Alternative methods comprise the recombinase-mediated integration of polynucleotides. For this purpose, the construct which is to be integrated is flanked by non-identical recombinase target sites such as loxP of lox2272 which are recognised by a recombinase, such as Cre recombinase allowing the insertion of the polynucleotide of the invention by homologous recombination in a chromosomal region flanked by loxP or lox2272 sites. Once the non-human transgenic animals carrying the constructs of the invention are obtained, it is necessary to identify those cells wherein a phenotypic alteration takes place. The expert will appreciate that the phenotypic alteration that is to be detected will depend on the type of biological process under study. Thus, if the process under study is the response to toxic agents, the phenotypic alteration to detect will be an increased resistance to said agents, if the process under study is cell proliferation, the phenotypic alterations will be, *inter alia,* an increase in the doubling rate, the capacity to overcome a chemically-induced mitotic block; if the process to study is the sensibility of a cell or organism to a pathogenic agent, the phenotypic alteration to detect would be an increase or decrease of the sensibility of the cell or organism to the infection by the pathogenic agent.

The type of phenotypic alterations that may be used for the detection of genes involved in a given biological process varies and depends essentially of the type of biological process that is to be studied. Thus, it is possible to detect morphological changes, biochemical changes (enzymatic activity, changes in the levels of one or more metabolites, changes in protein pattern obtained by 2D-gel, phosphorylated proteins),as well as behavioural changes (chemotropism, phototropism and the like).

Once the cells or organisms showing the desired phenotype have been identified, it is necessary to rescue the polynucleotide encoding the miRNA. This can be carried out by PCR from cell DNA or by genome viral rescue if the vector used for expressing the miRNA was a viral vector.

### Method for the identification of genes involved in a cellular process

The screening method of the invention also allows the identification of the genes involved in cellular function since, once a miRNA has been isolated which is capable of modulating a given cellular function, the sequence derived from the sense strand of the miRNA can be used to identify sequences within the genome showing substantial sequence homology either in the 3'-UTR if the miRNA is acting canonically by blocking translation or in the ORF if the miRNA is acting in a non-canonical way by promoting cleavage of the mRNA. Thus, in another aspect, the invention relates to a method for the identification of genes involved in a cellular process (hereinafter fourth method of the invention) comprising
(i) contacting a miRNA library as of the invention with a cell population under conditions adequate for promoting the entry of the library polynucleotides in the cell,
(ii) maintaining the cells under conditions adequate for promoting expression of the library-encoded miRNA,
(iii) selecting those cells showing an alteration in the cellular process under study,
(iv) recovering the library polynucleotides from the cell,
(v) sequencing the region of the library polynucleotide flanked by the promoter regions and
(vi) identifying genes showing a high degree of sequence relatedness in their 3'-UTR region with the sequence determined in step (v).

Steps (i) to (iv) are carried out essentially as described for the third method of the invention. Once the polynucleotide is recovered from the cell, the region corresponding to the sense and antisense strand of the miRNA is sequenced using standard methods.

Step (v) involves the sequence if the region of the library polynucleotide which is flanked by the promoter regions. The sequencing step can be carried out using any known means of sequencing such as chemical sequencing (Maxam-Gilbert), Sanger dideoxy sequencing, pyrosequencing, fluorescence detection sequencing and mass spectrometry DNA Sequencing

Step (vi) of the fourth method of the invention involves identifying sequences showing substantial similarity with the sequences determined in step (v). Preferably, the polynucleotide sequences flanked by the promoters are compared with miRNA databases to determine whether the different sequences are known or unknown miRNAs. A suitable miRNA database that can be interrogated with the sequences obtained in step (v) is, without limitation, the database located at http://microrna.sanger.ac.uk. If the sequences are not known or are not found in miRNA databases, then the sequences are compared with common polynucleotide databases. Adequate methods for determining the degree of sequence relatedness involve alignment of the sequences identified in step (v) with the sequences publicly available in the databases. Optimal alignment of sequences for comparison can be conducted, for example, by the local homology algorithm of Smith and Waterman (Adv. Appl. Math., 1981, 2:482), by the homology alignment algorithm of Needleman and Wunsch (J. MoI. Biol., 1970, 48:443-453), by the search for similarity method of Pearson and Lipman (Proc. Natl. Acad. Sci. USA, 1988, 85:2444-2448), by computerized implementations of these algorithms (e.g., GAP, BESTFIT, FASTA, and TFASTA in the Wisconsin Genetics Software Package, Genetics Computer Group, 575 Science Dr., Madison, Wis.), or by visual inspection. (See generally Ausubel et al. (eds.), Current Protocols in Molecular Biology, 4th ed., John Wiley and Sons, New York (1999)). Another example of an algorithm that is suitable for determining percent sequence identity and sequence similarity is the BLAST algorithm, which is described by Altschul et al. (J. MoI. Biol., 1990, 215:403-410) (See also Zhang et al., Nucleic Acid Res. 26:3986-90 (1998); Altschul et al., Nucleic Acid Res. 25:3389-402 (1997), which are incorporated by reference herein). The BLAST algorithm performs a statistical analysis of the similarity between two sequences (see, e.g., Karlin and Altschul, Proc. Natl. Acad. Sci. USA 90:5873-77 (1993), which is incorporated by reference herein). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.1, more typically less than about 0.01, and most typically less than about 0.001.

Optimal alignment of sequences for comparison can be conducted, for example, by the local homology algorithm of Smith and Waterman (Adv. Appl. Math., 1981, 2:482), by the homology alignment algorithm of Needleman and Wunsch (J. MoI. Biol., 1970, 48:443-453), by the search for similarity method of Pearson and Lipman (Proc. Natl. Acad. Sci. USA, 1988, 85:2444-2448), by computerized implementations of these algorithms (e.g., GAP, BESTFIT, FASTA, and TFASTA in the Wisconsin Genetics Software Package, Genetics Computer Group, 575 Science Dr., Madison, Wis.), or by visual inspection. (See generally Ausubel et al. (eds.), Current Protocols in Molecular Biology, 4th ed., John Wiley and Sons, New York (1999)). Another example of an algorithm that is suitable for determining percent sequence identity and sequence similarity is the BLAST algorithm, which is described by Altschul et al. (J. MoI. Biol., 1990, 215:403-410) (See also Zhang et al., Nucleic Acid Res. 26:3986-90 (1998); Altschul et al., Nucleic Acid Res. 25:3389-402 (1997), which are incorporated by reference herein). The BLAST algorithm performs a statistical analysis of the similarity between two sequences (see, e.g., Karlin and Altschul, Proc. Natl. Acad. Sci. USA 90:5873-77 (1993), which is incorporated by reference herein). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.1, more typically less than about 0.01, and most typically less than about 0.001.

Once a gene showing substantial homology to the miRNA has been identified, it is possible to confirm whether the miRNA is actually capable of inhibiting its expression.

The expression "Inhibition of gene expression" refers to the absence (or observable decrease) in the level of protein and/or mRNA product from a target gene. "Specificity" refers to the ability to inhibit the target gene without manifest effects on other genes of the cell. The consequences of inhibition can be confirmed by examination of the outward properties of the cell or organism (as presented below in the examples) or by biochemical techniques such as RNA solution hybridization, nuclease protection, Northern hybridization, reverse transcription, gene expression monitoring with a microarray, antibody binding, enzyme linked immunosorbent assay (ELISA), Western blotting, radioimmunoassay (RIA), other immunoassays, and fluorescence activated cell analysis (FACS). For RNA-mediated inhibition in a cell line or whole organism, gene expression is conveniently assayed by use of a reporter or drug resistance gene whose protein product is easily assayed. Such reporter genes include acetohydroxyacid synthase (AHAS), alkaline phosphatase (AP), beta galactosidase (LacZ), beta glucoronidase (GUS), chloramphenicol acetyltransferase (CAT), green fluorescent protein (GFP), horseradish peroxidase (HRP), luciferase (Luc), nopaline synthase (NOS), octopine synthase (OCS), and derivatives thereof. Multiple selectable markers are available that confer resistance to ampicillin, bleomycin, chloramphenicol, gentamycin, hygromycin, kanamycin, lincomycin, methotrexate, phosphinothricin, puromycin, and tetracyclin. In a preferred embodiment, a chimeric gene is constructed
wherein the coding sequence of a reporter gene is fused to a 3'-UTR comprising specific binding sites for the miRNA. If the miRNA leads to a decrease in the activity of the polypeptide encoded by the reporter gene without substantially affecting the mRNA levels, it is usually indicative that the miRNA is specific for any cellular mRNA carrying the above 3'-UTR.

The present invention is not limited to any type of target gene or nucleotide sequence that can be identified using the method of the invention. The following classes of possible target genes are listed for illustrative purposes: developmental genes (e.g., adhesion molecules, cyclin kinase inhibitors, Writ family members, Pax family members, Winged helix family members, Hox family members, cytokines/lymphokines and their receptors, growth/differentiation factors and their receptors, neurotransmitters and their receptors); oncogenes (e.g., ABLI, BCLI, BCL2, BCL6, CBFA2, CBL, CSFIR, ERBA, ERBB, EBRB2, ETSI, ETS1, ETV6, FGR, FOS, FYN, HCR, HRAS, JUN, KRAS, LCK, LYN, MDM2, MLL, MYB, MYC, MYCLI, MYCN, NRAS, PIM 1, PML, RET, SRC, TALI, TCL3, and YES); tumor suppressor genes (e.g., APC, BRCA 1, BRCA2, MADH4, MCC, NF 1, NF2, RB 1, TP53, and WTI); and enzymes (e.g., ACC synthases and oxidases, ACP desaturases and hydroxylases, ADP-glucose pyrophorylases, ATPases, alcohol dehydrogenases, amylases, amyloglucosidases, catalases, cellulases, chalcone synthases, chitinases, cyclooxygenases, decarboxylases, dextrinases, DNA and RNA polymerases, galactosidases, glucanases, glucose oxidases, granule-bound starch synthases, GTPases, helicases, hemicellulases, integrases, inulinases, invertases, isomerases, kinases, lactases, lipases, lipoxygenases, lysozymes, nopaline synthases, octopine synthases, pectinesterases, peroxidases, phosphatases, phospholipases, phosphorylases, phytases, plant growth regulator synthases, polygalacturonases, proteinases and peptidases, pullanases, recombinases, reverse transcriptases, RUBISCOs, topoisomerases, and xylanases).

### Methods for the identification of small RNAs capable of interfering with a cellular process and genes involved in said process

The authors of the present invention have also observed that the method described herein allows not only the cloning and characterization of miRNAs present in an RNA preparation, but also allows the direct expression of the cloned molecules. In the methods known in the prior art, once the small RNA was identified, it had to be sequenced and, once the sequence was available, the corresponding cDNA had to be obtained so that it could be used as template for producing the small RNA from a single promoter. The inventors have found that it is possible to express functional miRNAs from the vectors wherein they are cloned if convergent promoter regions are conveniently placed in the cloning vector. While this observation is particularly interesting for miRNAs, it is equally suitable to any double stranded RNA. Thus, the method can be used for the cloning of small RNA molecules as well as for the identification of small RNA molecules involved in a given cellular process without the need of sequencing every potential RNA prior to its functional testing.

Thus, in another aspect, the invention relates to a method (hereinafter, the fifth method of the invention) for the cloning of small RNAs comprising the steps of
(i) contacting a RNA preparation having about 19-25 nucleotides in length with a 5' adapter and a 3' adapter of known sequences, wherein said contacting step is carried out under conditions adequate for promoting ligation of the adapters to the 5' and 3' ends of the RNA and for preventing circularization of the RNA,
(ii) reverse transcribing the 5'-adapter-RNA-3'adapter molecules obtained in step (i) using a primer which sequence is substantially complementary to the sequence of the 3' adapter,
(iii) producing a double stranded polynucleotides using as template the cDNAs obtained in step (ii) and
(iv) inserting the polynucleotides obtained in step (iii) in a suitable vector
wherein convergent promoter sequences are present either within the known sequences of the 5' and 3' adapters used in step (i) or in the vector wherein the polynucleotide is inserted in step (iv) at positions 5' or 3 with respect of the site of insertion.

In yet another aspect, the invention relates to a method (hereinafter, the sixth method of the invention) for the identification of small RNAs involved in a biological process comprising the steps of
(i) contacting a RNA preparation having about 19-25 nucleotides in length with a 5' adapter and a 3' adapter of known sequences, wherein said contacting step is carried out under conditions adequate for promoting ligation of the adapters to the 5' and 3' ends of the RNA and for preventing circularization of the RNA,
(ii) reverse transcribing the 5'-adapter-RNA-3'adapter molecules obtained in step (i) using a primer which sequence is substantially complementary to the sequence of the 3' adapter,
(iii) producing a double stranded polynucleotides using as template the cDNAs obtained in step (ii),
(iv) inserting the polynucleotides obtained in step (iii) in a suitable vector,
(v) contacting the polynucleotides obtained in step (iv) with a cell population under conditions adequate for promoting the entry of the library polynucleotides in the cell,
(vi) maintaining the cells under conditions adequate for promoting expression of the library-encoded small RNAs,
(vii) selecting those cells showing an alteration in the cellular process under study and
(viii) recovering the library polynucleotides from the cell.
wherein convergent promoter sequences are present either within the known sequences of the 5' and 3' adapters used in step (i) or in the vector wherein the polynucleotide is inserted in step (iv) at positions 5' or 3 with respect of the site of insertion.

The term "RNA population", as used herein, relates to any collection of RNA either isolated from a natural source or obtained *in vitro.* While the method of the invention was initially designed for the identification of miRNAs, the authors of the invention have observed that, surprisingly, the method is also suitable for any type of endogenous double stranded RNA. This includes, without limitation:
- miRNAs,
- the recently identified mouse siRNAs which are generated from dsRNAs derived from repeat sequences most of them from retrotrasposons and pseudogenes (Watanabe et al, 2008, Nature, 453:539-43 and Tam et al, 2008,. Nature, 453:534-8),
- piRNA or Piwi interacting RNA are 24- to 30-nt RNAs that are generated by a Dicer-independent mechanism and that interact with a subset of Argonaute proteins related to Piwi (Klattenhof & Theurkauf, 2008, Development, 135:3-9). Different studies in mice, *Drosophila* and Zebrafish have demonstrated that these piRNAs are involved in germline development. In addition, proteins engaged in piRNA production have been related to the control of gene expression in somatic cells, suggesting a broad range impact in several biological processes.
- snoRNAs-miRNA like (Ender et al, 2008, Mol Cell 32:519-528), which have become important in different biological processes.

Steps (i) to (iv) of the fifth and sixth method of the invention are essentially carried out as described previously in the fourth method for the preparation of miRNA libraries. In this method, it is important that the initial RNA preparation has not been treated so as to remove RNA molecules different from miRNA, since it would be equally suitable for the identification of siRNA, piRNA and the like. Preferably, the initial RNA preparation has been obtained from a cell using any of the methods previously described.

Steps (v) to (viii) of the sixth method of the invention are then carried out essentially as described before for the methods for the identification of a miRNA relevant for a biological process and include the contacting of the library obtained in step (iv) with a target cell, allowing the small RNAs encoded by the library polynucleotides to be expressed by the cell, identifying those cells showing a phenotypic change and recovering the library polynucleotide from the phenotypically changed cell.

In a preferred embodiment, the polynucleotide recovered by the fifth method of the invention is selected from the group of miRNA, endogenous siRNA, piRNA and snoRNAs-miRNA like.

The RNAs identified by the sixth method of the invention are also useful for the identification of the target genes, since it is to be expected that the small RNAs will show a certain degree of sequence identity with the target genes. Thus, in another aspect, the invention relates to a method (hereinafter the seventh method of the invention) for the identification of genes involved in a cellular process comprising
(i) contacting a RNA preparation having about 19-25 nucleotides in length with a 5' adapter and a 3' adapter of known sequences,
   wherein said contacting step is carried out under conditions adequate for promoting ligation of the adapters to the 5' and 3' ends of the RNA and for preventing circularization of the RNA,
(ii) reverse transcribing the 5'-adapter-RNA-3'adapter molecules obtained in step (i) using a primer which sequence is substantially complementary to the sequence of the 3' adapter,
(iii) producing a double stranded polynucleotides using as template the cDNAs obtained in step (ii),
(iv) inserting the polynucleotides obtained in step (iii) in a suitable vector
(v) contacting the polynucleotides obtained in step (iv) with a cell population under conditions adequate for promoting the entry of the library polynucleotides in the cell,
(vi) maintaining the cells under conditions adequate for promoting expression of the library-encoded small RNAs,
(vii) selecting those cells showing an alteration in the cellular process under study,
(viii) recovering the library polynucleotides from the cell,
(ix) sequencing the region of the library polynucleotide flanked by the promoter regions and
(x) identifying genes showing a high degree of sequence relatedness with the sequence determined in step (v)
wherein convergent promoter sequences are present either within the known sequences of the 5' and 3' adapters used in step (i) or in the vector wherein the polynucleotide is inserted in step (iv) at positions 5' or 3 with respect of the site of insertion.

Steps (i) to (iv) of the seventh method of the invention are carried out essentially as described in steps (i) to (iv) of the method for the preparation of a miRNA library of the invention. In a preferred embodiment, the population of miRNA molecules has been obtained from a cell.

Steps (v) to (x) of the seventh method of the invention are carried out essentially as described in steps (i) to (vi) of the method for the identification of genes which are involved in a cellular process. However, whereas the method described above is limited to the identification of the sequences of the library miRNAs in the 3'-UTR of a gene, the method described herein is not limited to the identification of the relevant sequences within the 3'-UTR but can be carried out all through the length of the target gene. It will be appreciated that the method will lead to the cloning in step (iv) of any type of small RNA present in the cell. Thus, in a preferred embodiment, the method further comprises determining the type of small RNA encoded by the polynucleotides recovered in step (viii). Preferably, the polynucleotide sequences flanked by the promoters are compared with miRNA databases to determine whether the different sequences are known or unknown miRNAs. A suitable miRNA database that can be interrogated with the sequences obtained in step (ix) is, without limitation, the database located at http://microrna.sanger.ac.uk.

In a preferred embodiment, the 3' end of the 3' adapter used in step (i) is protected. In a more preferred embodiement, the protection of the 3' end of the 3' adaptar is a dideoxy-C group. In another preferred embodiment, the 5' end of the 3' adapter is adenylated. In a still more preferred embodiment, the 5' adapter is an unphosphorylated sequence which 3' region comprises one or more ribonucleotides.

### Kits of the invention

In yet another aspect, the invention relates to a kit for carrying out the methods as defined previously comprising
(i) a 5' adapter comprising a first known sequence,
(ii) a 3' adapter comprising a second known sequence,
(iii) a vector comprising at least a cloning site,
(iv) a RNA ligase and
(v) a reverse transcriptase
wherein promoter sequences are present either within the first and second known sequences of the adapters or in the vector 5' and 3' with respect to the cloning site.

The adapters forming part of the kit have been defined previously and comprise, preferably, a 3' adapter protected protected at the 3'end and, more preferably, protected by a dideoxy-C group. In yet another embodiment, the 3' adapter contains an adenylated 5' end. Suitable reverse transcriptases and RNA ligases have been described before in the context of the first method of the invention.

The kit may also contain components needed for further processing of the cDNA obtained with components (i) to (iv). Thus, the kit may also contain reagents adequate for producing a second strand cDNA such as one or more primers, a DNA polymerase, dNTPs, pyrophosphatase and the like.

In a preferred embodiment, the 5' and 3' adapter comprise target sites for a restriction endonuclease which is placed 5' with respect to the promoter region. The endonuclease target sites may be identical or different. If the adapters contain endonuclease target sites, the kit may also contain the endonuclease or endonucleases suitable for digesting the resulting double-stranded polynucleotides. Suitable restriction endonucleases have been described before in the context of the first method of the invention.

Moreover, the kit may also comprise a vector suitable for cloning the polynucleotides encoding the miRNA. The vector may be circular or may be pretreated with a restriction endonuclease to allow insertion of the digested polynucleotide. If the kit comprises a vector, then it is also convenient to supply a DNA ligase.

Kits according to this aspect of the invention may comprise one or more containers, which may contain one or more components selected from the group consisting of one or more nucleic acid molecules or vectors, one or more primers, supports, one or more polymerases, one or more reverse transcriptases, one or more recombination proteins (or other enzymes for carrying out the methods of the invention), one or more buffers, one or more detergents, one or more restriction endonucleases, one or more nucleotides, one or more terminating agents (e.g., ddNTPs), one or more transfection reagents, pyrophosphatase, and the like.

A wide variety of nucleic acid molecules or vectors of the invention can be used with the invention. Further, due to the modularity of the invention, these nucleic acid molecules and vectors can be combined in wide range of ways. Examples of nucleic acid molecules which can be supplied in kits of the invention include those that contain promoters, signal peptides, enhancers, repressors, selection markers, transcription signals, translation signals, primer hybridization sites (e.g., for sequencing or PCR), recombination sites, restriction sites and polylinkers, sites which suppress the termination of translation in the presence of a suppressor tRNA, suppressor tRNA coding sequences, sequences which encode domains and/or regions (e.g., 6 His tag) for the preparation of fusion proteins, origins of replication, telomeres, centromeres, and the like. Similarly, libraries can be supplied in kits of the invention. These libraries may be in the form of replicable nucleic acid molecules or they may comprise nucleic acid molecules which are not associated with an origin of replication. As one skilled in the art would recognize, the nucleic acid molecules of libraries, as well as other nucleic acid molecules, which are not associated with an origin of replication either could be inserted into other nucleic acid molecules which have an origin of replication or would be an expendable kit components.

It will be appreciated that the kit may also contain one or more buffers for carrying out the methods of the invention, i.e. a RNA ligation buffer for ligating the adapters, a reverse transcription buffer, a second strand syntesis buffer, a PCR buffer, a DNA ligation buffer and the like. These buffers may be supplied at a working concentrations or may be supplied in concentrated form and then diluted to the working concentrations. These buffers will often contain salt, metal ions, co-factors, metal ion chelating agents, etc. for the enhancement of activities of the stabilization of either the buffer itself or molecules in the buffer. Further, these buffers may be supplied in dried or aqueous forms. When buffers are supplied in a dried form, they will generally be dissolved in water prior to use.

Kits of the invention may contain virtually any combination of the components set out above or described elsewhere herein. As one skilled in the art would recognize, the components supplied with kits of the invention will vary with the intended use for the kits. Thus, kits may be designed to perform various functions set out in this application and the components of such kits will vary accordingly. The kit may also contain instructions for carrying out the methods of the invention. Said instructions can be in the form of printed material or in the form of an electronic medium capable of storing instructions so that they can be read by a subject, such as electronic storage media (magnetic disks, tapes and the like), optical media (CD-ROM, DVD) and the like. Additionally or alternatively, the media can contain internet links which provide said instructions.

The invention is explained below by the following examples which are to be construed as merely illustrative and not limitative of the scope of the invention.

### EXAMPLES

### The lentiviral vector

The modified lentiviral vector Lent-H1/U6 contains two terminal RNA polymerase III sequences (T5) just upstream of the transcriptional star sites of H1 and U6 promoters.

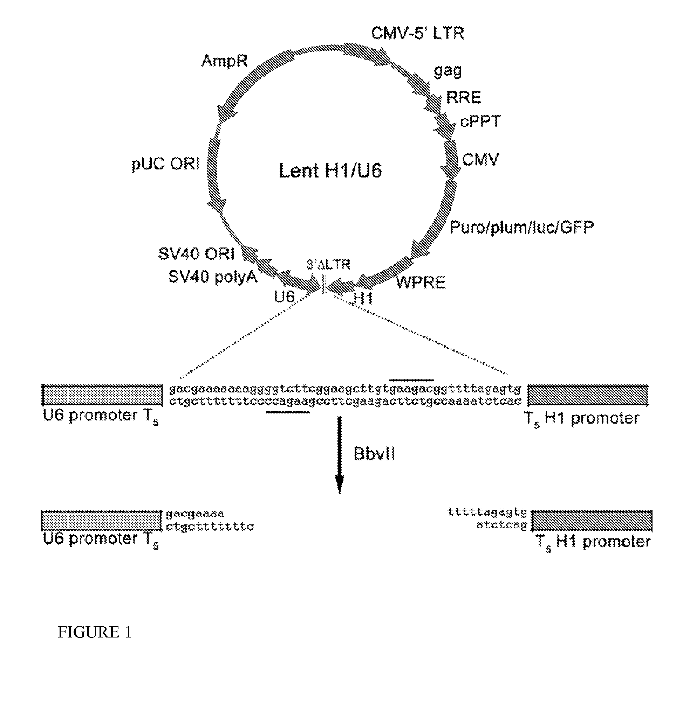

Figure 1 shows the map of the lentiviral vector and its digestion with Bbvll restriction enzyme, that recognizes the sequence GAAGACN2/N6.

The appropiate marker in the lentiviral vector depends on the specific experiment. Originally, the vector contained a puromycin resistance gene, subcloned between the Xbal and SalI restriction sites. This gene has been replaced by the markers EGFP, mPlum and Luciferase, released respectively from pEGFP-N1 vector (Clontech), pmPlum vector (provided by Dr. Roger Y.Tsien) and pGL4.10 vector (Promega). mPlum encodes a mutant fluorescent protein derived from the tetrameric Discosoma sp. red fluorescent protein, DsRed. The maximal excitation and emission wavelength are 590 nm and 649 nm respectively. This makes this protein suitable as a marker for in vivo imaging, because animal tissues absorb poorly this fluorescence emission. The luciferase gene from pGL4.10 vector contains a single restriction site for BbvII, and its codons have been optimized for expression in mammalian cells. Luciferase codons 18 (GAA) and 19 (GAG) contain the BbvII restriction site; this has been destroyed by mutagenesis of codon 18 (GAABAG), using the oligonucleotide 5'-GCCATTCTACCCACTCGAGGACGGGACCGCCGGCGAGCAGC-3' (SEQ ID NO:1) and the Multi Site-Directed Mutagenesis Kit (Stratagene).

After transcription, the resulting miRNA has the same structure as the "natural" double-stranded processed miRNA and does not require the cleavage steps. Moreover, the double promoter Lent-H1/U6 vector provides higher stability of RNA template inserts for propagation in *E.coli.* This feature is critical for the construction of representative high complexity miRNA libraries.

### Validation of the lentiviral vector for expressing of miRNAs

Experiments were carried out to demonstrate that the expression of short miRNAs is functionally similart to the full lenght versions. For this purpose, processed sequences of the miRNA-21 (mir-21), miRNA-19 (mir-19) and miRNA-15 (mir-15) were cloned into the Lent-H1/U6 vector.

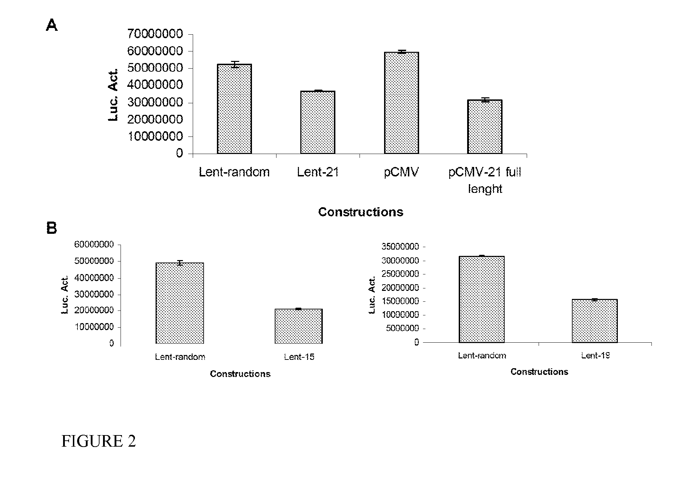

The functionality of the Lent-H1/U6-miRNA system to suppress gene expression was examined using the luciferase gene containing the specific miRNA binding site (BS) sequence in its 3' UTR region (pCDNA3.1::LUC::BS mir21, mir15 or mir19). The activity of the miRNA has been measured in luciferase assays. HEK 293T cell line was selected because they lack these miRNAs or express them at very low levels. HEK 293T cells were co-transfected with Lent-H1/U6-miRNA21, pCDNA::LUC::BSmir21 and pCMV-Renilla plasmids at 100:10:1 proportion. Moreover, a Lent-H1/U6random (with a random sequence) was used as negative control. pCMV::mir21 full length plasmid was used as a positive control of the experiment. When processed or full length version of mir21 was used, very similar results were observed and depletion of 35-40% of luciferase activity could be measured (figure 2A). Moreover, this luciferase activity depletion was observed in similar experiments using Lent-H1/U6- miRNA19 or 15 plasmids (figure 2B). These experiments demonstrate the general applicability of Lent-H1/U6-miRNA to drive functional miRNA expression experiments.

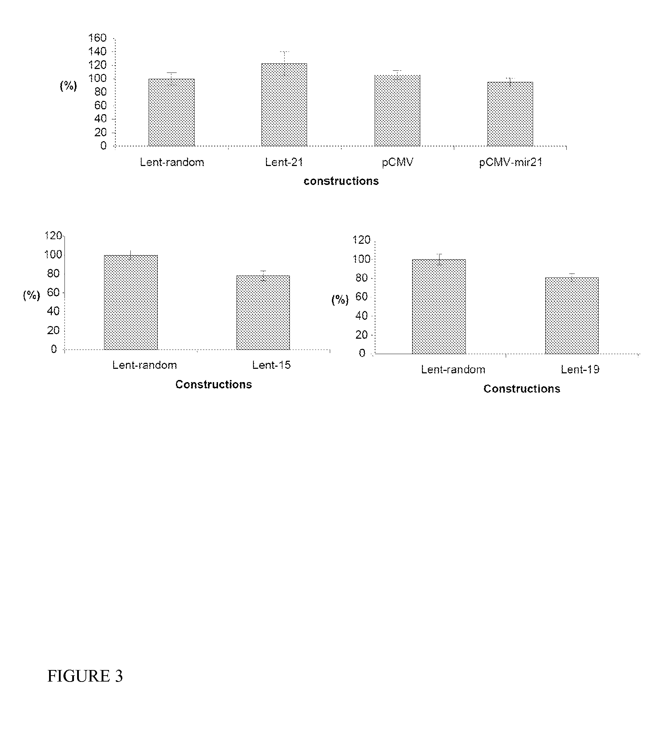

Because miRNAs work depleting translation, a Real Time RT-PCR experiment was performed to demonstrate that Lent constructions produce a decrease of luciferase activity by blocking the translation instead degradation of RNA (Figure 3).

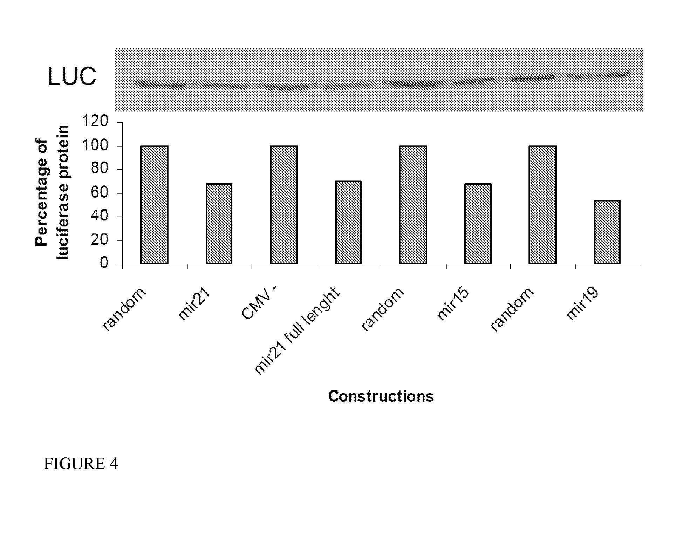

In fact, a decrease of luciferase protein levels (30-35%) could be observed in cells transfected with Lent-miR constructions when cell extracts were analysed by Western blot using luciferase-specific antiboies (figure 4). All these results demonstrate that the expression system of mature miRNA is functionally similar to the full length miRNA expression system.

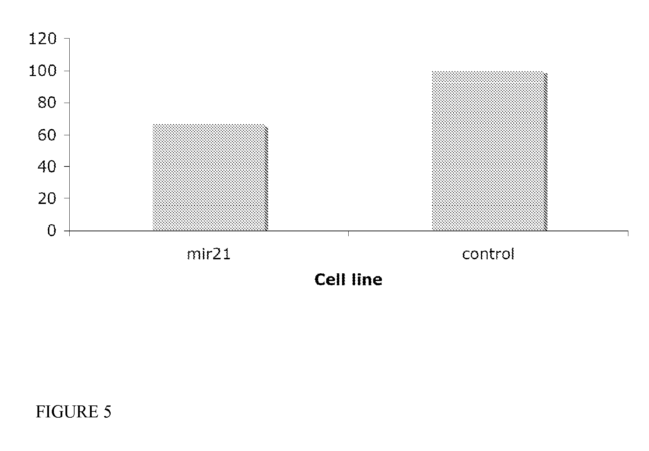

On the other hand, expression of these proccesed miRNAs has been determined using an RNase protection assay with probes designed to identify both transcripts. Radiolabeled RNA probes was generated with the "mirVana mRNA probe construction kit" (Ambion) and it was demonstrated that only sense transcripts were expressed and responsible for the luciferase depletion (data not shown). Moreover, the processed miRNA-21 knocked down a natural target of the full length version, PTEN protein (figure 5) (Meng et al., 2007, Gastroenterology, 133:647-58). All these results demonstrate that the system of expression of processed miRNAs are functionally similar to the full length and can be employed to different applications.

### Generation of miRNAs libraries

As previously mentioned, the design of the miRNAs libraries is based on the transcription of both strands of a short DNA fragment flanked by two RNA polymerase III promoters, generating a duplex of RNA.

The generation of miRNA libraries comprises the following steps
1) Isolation of small RNAs of a size of 19-25 nucleotides by polyacrylamide gel electrophoresis.
2) Synthesis of the 5' phophorimidazolide of adenosine (ImpA), followed by chemical adenylation of the 5' phosphate of the 3'-adapter oligonucleotide using ImpA. As described by Lohrmann and Orgel (Lohrmann & Orgel, 1978, J Mol Evol, 11:17-23).
3) Adding a 3'-terminal dideoxy-C to the adenylated 3' adapter to obtain an adapter having the sequence AppTTTTTTGTCTTCCGCCCGTCGCCATAddC (SEQ ID NO:2)
4) Syntehsis of the 5'Adapter as a hybrid unphosphorylated sequence having the sequence 5'-CGGCTCAACTCTCTGGAAGActcaaag-3' (SEQ ID NO:3), wherein lower-cases represent ribonucleotides. Both adapters contain a Bbv11 restriction site and were obtained for priming reverse transcription and for defining the orientation of the cloned small RNA.
5) Ligation of the adenylated 3' adapter to small RNAs by T4 RNA ligase in the absence of ATP. This condition prevents circularization of the 5' phosphorylated small RNAs.
6) Ligation of the 5' adapter in presence of ATP and T4 RNA ligase
7) Reverse transcripion of the final ligation product using the primer 5'-GTATGCCCGCTCAGTGAA-3' (SEQ ID NO:4) which corresponds to a sequence found in the adapters
8) RNA hydrolysis followed by amplification of the oligonucleotide using the biotinylated primers 5'-GTATGCCCGCTCAGTGAA-3' (SEQ ID NO;5) and 5' CGGCTCAACTCTCTGGAA-3' (SEQ ID NO:6).
9) Purification of the amplified product in a polyacrylamide gel electrophoresis and digestion with BbvII endonuclease.
10)Elution and precipitation of DNA without biotin the using a Streptavidin-biotin affinity system
11)Ligation of the purified fragments to the BbvII-digested Lent-H1/U6 vector.
12)Transformation of electro competent DH5a bacteria
13)Recovery of the Lent-H1/U6 plasmids Lent-H1/U6 from recombinant colonies.
14)Preparation of high titer viral preparation by cotransfection of the Lent-H1/U6 plasmid with the packaging vectors Lent-34N and pVSV-G into the packaging cells HEK293T.
15) Titration of the lentivirus in HEK 293T cells using the specific selection marker.

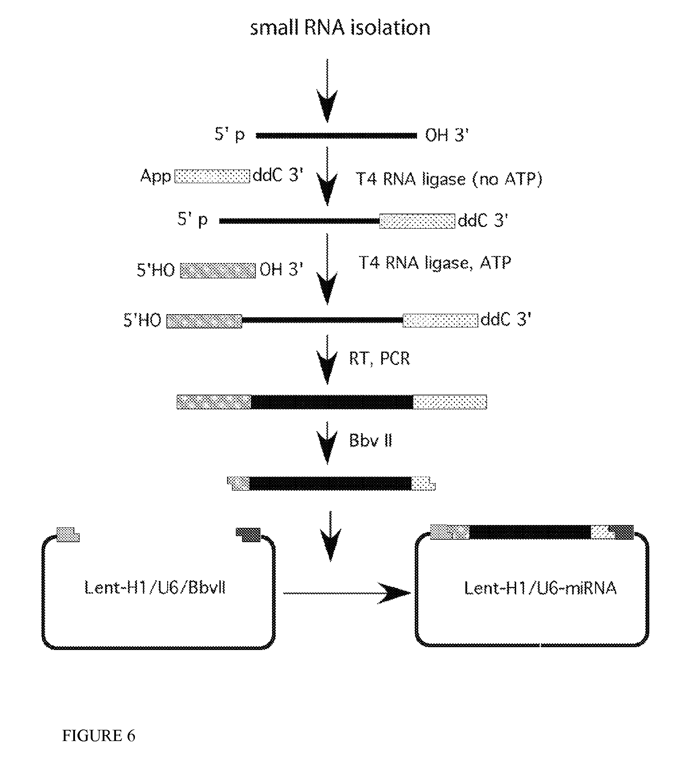

The synthesis scheme is summarized in figure 6. The library obtained according to the previous protocol can be used directly in every specific functional screening.

One of the advantages of lentiviral-based systems is the integration of proviral DNA in the genome of the cell. This feature makes possible to isolate the DNA fragment corresponding to the miRNA cloned into the lentivirus. Genomic DNA isolated can be amplified with the H1 (5'-CTGGGAAATCACCATAAACGTGAA-3') (SEQ ID NO:7) and U6 (5'- GCTTACCGTAACTTGAAAGTATTTCG-3') (SEQ ID NO:8) primers. PCR products can be subcloned and sequenced.

Identification of genes corresponding to isolated miRNAs or other types of smallRNAs is carried out as follows. Firstly, it is determined whether the isolated smallRNAs are cathaloged in the miRNA data base (http://www.sanger.ac.uk/Software/Rfam/mirna). In a negative case, the putative miRNA that produces a specific phenotype can be identified in the genome and flanking sequences can be analyzed. There are several softwares (Berezikov et al, 2006, Nature Genetics, 38: S2-S7) to determinate whether a specific sequence can be a miRNAs. Those sequences with a high probability to be a real miRNAs, can be expressed in a full lenght version. If the overexpression of full length putative miRNA produces the same phenotype of proccesed version, a novel miRNA could be identified. Others methods and approaches to validate predicted candidate miRNAs are described (Berezikov et al, 2006, *supra.*) and all of them can be used to confirm that the results obtained in the screening are correct.

Using this system of isolation of miRNAs, several miRNA have been identified (such as mir21, mir34) from MDA-MB-231 (clone 4175) cells and others sequences that could be novel miRNAs.

RNA sequences from 19 to 25 nucleotides were cloned into a specific vector as described before and expressed to look for specific phenotypes. Once the screening have been performed, different sequences will be associated with these phenotypes and described and novels miRNA could be identified. One of the advantages of this system is the possibility of isolate, by a functional screening, new miRNAs which will have to be validate using different approaches.

## Claims

1. A polynucleotide construct comprising a first promoter region operatively linked to a sequence encoding the sense strand of a miRNA and a second promoter region operatively linked to a sequence encoding the antisense strand of said miRNA.

2. A polynucleotide as defined in claim 1 wherein the polynucleotide is a double stranded DNA and wherein the sequence encoding the sense strand forms a duplex with the sequence encoding the antisense strand.

3. A vector comprising a polynucleotide as defined in claims 1 or 2.

4. A library comprising a plurality of polynucleotides as defined in claims 1 or 2 or a plurality of vectors as defined in claim 3.

5. A host cell comprising a polynucleotide as defined in claims 1 or 2, a vector as defined in claim 3 or a library as defined in claim 4.

6. A method for expressing a miRNA in a host cell comprising contacting the cell with a polynucleotide as defined in claims 1 or 2, with a vector as defined in claim3 or with a library as defined in claim 4 under conditions adequate for entry of the polynucleotide or the vector in the cell and for the expression of said polynucleotide in the cell.

7. A method for the preparation of a vector for expressing a miRNA comprising the steps of
(i) contacting a miRNA molecule with a 5' adapter and a 3' adapter
wherein each adapter comprises a known sequence, wherein said contacting step is carried out under conditions adequate for promoting ligation of the adapters to the 5' and 3' ends of the RNA and for preventing circularization of the RNA and
(ii) reverse transcribing the 5'-adapter-RNA-3'adapter molecule obtained in step (i) using a primer which sequence is substantially complementary to the sequence of the 3' adapter and
(iii) producing a double stranded polynucleotide using as template the cDNA obtained in step (ii) and
(iv) inserting the polynucleotide obtained in step (iii) in a suitable vector
wherein convergent promoter sequences are present either within the known sequences of the 5' and 3' adapters or in the vector wherein the polynucleotide is inserted in step (iv) at positions 5' or 3 with respect of the site of the insertion.

8. A method for preparing a library for expressing a miRNA comprising the steps of
(i) contacting a RNA preparation comprising at least one miRNA molecule having about 19-25 nucleotides in length with a 5' adapter and a 3' adapter of known sequences, wherein said contacting step is carried out under conditions adequate for promoting ligation of the adapters to the 5' and 3' ends of the RNA and for preventing circularization of the RNA and
(ii) reverse transcribing the 5'-adapter-RNA-3'adapter molecules obtained in step (i) using a primer which sequence is substantially complementary to the sequence of the 3' adapter,
(iii) producing a double stranded polynucleotides using as template the cDNAs obtained in step (ii) and
(iv) inserting the polynucleotide obtained in step (iii) in a suitable vector
wherein convergent promoter sequences are present either within the known sequences of the 5' and 3' adapters or the vector wherein the polynucleotide is inserted in step (iv) at positions 5' or 3 with respect of the site of insertion.

9. A method as defined in claim 8 wherein the population of miRNA molecules has been obtained from a cell.

10. A method for the identification of miRNAs capable of interfering in a cellular process comprising
(i) contacting a miRNA library as defined in claim 8 with a cell population under conditions adequate for promoting the entry of the library polynucleotides in the cell,
(ii) maintaining the cells under conditions adequate for promoting expression of the library-encoded miRNAs
(iii) selecting those cells showing an alteration in the cellular process under study and
(iv) recovering the library polynucleotides from the cell.

11. A method for the identification of genes involved in a cellular process comprising
(i) contacting a miRNA library as defined in claim 8 with a cell population under conditions adequate for promoting the entry of the library polynucleotides in the cell,
(ii) maintaining the cells under conditions adequate for promoting expression of the library-encoded miRNA
(iii) selecting those cells showing an alteration in the cellular process under study,
(iv) recovering the library polynucleotides from the cell
(v) sequencing the region of the library polynucleotide flanked by the promoter regions and
(vi) identifying genes showing a high degree of sequence relatedness in their 3'-UTR region with the sequence determined in step (v).

12. A method for the cloning of small RNAs comprising the steps of
(i) contacting a RNA preparation having about 19-25 nucleotides in length with a 5' adapter and a 3' adapter of known sequences, wherein said contacting step is carried out under conditions adequate for promoting ligation of the adapters to the 5' and 3' ends of the RNA and for preventing circularization of the RNA,
(ii) reverse transcribing the 5'-adapter-RNA-3'adapter molecules obtained in step (i) using a primer which sequence is substantially complementary to the sequence of the 3' adapter,
(iii) producing a double stranded polynucleotides using as template the cDNAs obtained in step (ii) and
(iv) inserting the polynucleotides obtained in step (iii) in a suitable vector
wherein convergent promoter sequences are present either within the known sequences of the 5' and 3' adapters used in step (i) or in the vector wherein the polynucleotide is inserted in step (iv) at positions 5' or 3 with respect of the site of insertion.

13. A method for the identification of small RNAs involved in a biological process comprising the steps of
(i) contacting a RNA preparation having about 19-25 nucleotides in length with a 5' adapter and a 3' adapter of known sequences, wherein said contacting step is carried out under conditions adequate for promoting ligation of the adapters to the 5' and 3' ends of the RNA and for preventing circularization of the RNA,
(ii) reverse transcribing the 5'-adapter-RNA-3'adapter molecules obtained in step (i) using a primer which sequence is substantially complementary to the sequence of the 3' adapter,
(iii) producing a double stranded polynucleotides using as template the cDNAs obtained in step (ii),
(iv) inserting the polynucleotides obtained in step (iii) in a suitable vector,
(v) contacting the polynucleotides obtained in step (iv) with a cell population under conditions adequate for promoting the entry of the library polynucleotides in the cell,
(vi) maintaining the cells under conditions adequate for promoting expression of the library-encoded small RNAs,
(vii) selecting those cells showing an alteration in the cellular process under study and
(viii) recovering the library polynucleotides from the cell.
wherein convergent promoter sequences are present either within the known sequences of the 5' and 3' adapters used in step (i) or in the vector wherein the polynucleotide is inserted in step (iv) at positions 5' or 3 with respect of the site of insertion.

14. A method for the identification of genes involved in a cellular process comprising
(i) contacting a RNA preparation having about 19-25 nucleotides in length with a 5' adapter and a 3' adapter of known sequences,
wherein said contacting step is carried out under conditions adequate for promoting ligation of the adapters to the 5' and 3' ends of the RNA and for preventing circularization of the RNA,
(ii) reverse transcribing the 5'-adapter-RNA-3'adapter molecules obtained in step (i) using a primer which sequence is substantially complementary to the sequence of the 3' adapter,
(iii) producing a double stranded polynucleotides using as template the cDNAs obtained in step (ii),
(iv) inserting the polynucleotides obtained in step (iii) in a suitable vector
(v) contacting the polynucleotides obtained in step (iv) with a cell population under conditions adequate for promoting the entry of the library polynucleotides in the cell,
(vi) maintaining the cells under conditions adequate for promoting expression of the library-encoded small RNAs,
(vii) selecting those cells showing an alteration in the cellular process under study,
(viii) recovering the library polynucleotides from the cell,
(ix) sequencing the region of the library polynucleotide flanked by the promoter regions and
(x) identifying genes showing a high degree of sequence relatedness with the sequence determined in step (v)
wherein convergent promoter sequences are present either within the known sequences of the 5' and 3' adapters used in step (i) or in the vector wherein the polynucleotide is inserted in step (iv) at positions 5' or 3 with respect of the site of insertion.

15. A method as defined in any of claims 12 to 14 wherein the population of miRNA molecules has been obtained from a cell.

16. A method as defined in any of claims 12 to 15 further comprising determining the type of small RNA encoded by the polynucleotides recovered in step (viii).

17. A kit for carrying out the method as defined in any of claims 10 to 16 comprising
(i) a 5' adapter comprising a first known sequence,
(ii) a 3' adapter comprising a second known sequence,
(iii) a vector comprising at least a cloning site,
(iv) a RNA ligase and
(v) a reverse transcriptase
wherein promoter sequences are present either within the first and second known sequences of the adapters or in the vector 5' and 3' with respect to the cloning site.
